# EUROPEAN PATENT APPLICATION

(11) **EP 3 922 642 A1**
(43) Date of publication of application: **15.12.2021**
(21) Application number: 20752216.0
(22) Date of filing: 07.02.2020
(51) Int. Cl.: C07K 14/47, C12N 5/0781, A61K 35/17

(54) **METHOD FOR T CELL ACTIVATION FOR CANCER TREATMENT**

(30) Priority: 08.02.2019 KR 20190015108
(71) Applicant: Good T Cells, Inc., Seoul 03722 (KR)
(72) Inventor: KIM, Jung Ho, Seoul 04136 (KR); KIM, Beom Seok, Seoul 04029 (KR)
(74) Representative: Schnappauf, Georg
(86) International application number: PCT/KR2020/001734
(87) International publication number: WO 2020/162696

(57) **Abstract**

The antigen-presenting cell loaded with the cancer-specific tumor antigen epitope provided in the present invention, that is, a dendritic cell enables rapid and effective induction of differentiation and proliferation of cancer antigen-specific T cells, preferably memory T cells, and the memory T cells thus activated can treat a cancerous or neoplastic condition or prevent recurrence, progression, or metastasis of cancer while avoiding the defense mechanism of cancer cells.

## Description

### Technical Field

The present invention relates to a cancer-specific tumor antigen epitope, an antigen-presenting cell loaded with the epitope, and a method for activating T cells for cancer treatment using the antigen-presenting cell.

### Background Art

Gastric cancer is known as a malignancy with a high incidence in the world, especially in Asia. There have been many known causes of development of gastric cancer; however, gastric cancer may be typically classified into EBV-associated gastric cancer, which is caused by infection with Epstein-Barr virus (EBV), and gastric cancer cell antigen-associated gastric cancer, which is caused by accumulation of genetic mutations in gastrointestinal cells. For current treatment for gastric cancer, excision of cancerous tissue has long been known to be the most effective, and chemotherapy and radiation therapy are also performed. However, it appears that gastric cancer is a hard-to-cure disease when not found early. In addition, although clinical trials have been conducted through several biological agents (antibodies, small molecules), therapeutic agents with good clinical effects have not yet been reported.

Recently, cancer cell-specific targeted therapy using patient-derived autologous T cells has been studied in several institutions, and clinical trials have been conducted for lymphoma using chimeric antigen receptor (CAR) T cells in several institutions. As a result, due to good clinical effects and low side effects, such therapy has attracted much attention as a new field of anticancer therapy.

Use of patient-derived T cells decreases induction of immune responses, which is the biggest side effect of cell therapeutic agents, and removes restrictions on the donor's HLA type. Thus, such T cells have been known as therapeutic agents which are effective and have no side effects. To date, CD8+ T cells, CD4+ T cells, NK cells, dendritic cells, and CAR T cells are known as types of cell therapeutic agents which are most widely used in the field of anticancer therapy. NK cells have cell-killing efficacy, and have several side effects due to not having antigen specificity. Dendritic cells are therapeutic agents belonging to the vaccine concept in that they have no function of directly killing cells, and are capable of delivering antigen specificity to T cells in the patient's body so that cancer cell specificity is imparted to T cells with high efficiency. In addition, CD4+ T cells play a role in helping other cells through antigen specificity, and CD8+ T cells are known to have the best antigen specificity and cell-killing effect.

However, most cell therapeutic agents, which have been used or developed to date, have limitations and thus have no clinical effect. Specifically, cancer cells, on their own, secrete substances that suppress immune responses in the human body, or do not present antigens necessary for production of antibodies against such cancer cells, thereby preventing an appropriate immune response from occurring.

Meanwhile, dendritic cells not only act as surveillants to detect antigens that come from the outside of the human body or are produced internally, but also quickly travel to the secondary lymphoid organs with such recognized and absorbed antigen, thereby acting as specialized antigen-presenting cells that present the antigens to immune cells, including T cells, which react with the antigens. Anti-cancer immunotherapeutic vaccines using dendritic cells have been developed through several methods, and may be largely divided into *ex vivo* generated dendritic cell vaccines and *in vivo* dendritic cell vaccines. The *in vivo* dendritic cell vaccine works in a manner of directly delivering an antigen to dendritic cells present in the body. In addition, a method using the *ex vivo* generated dendritic cell vaccine is in such a manner that dendritic cells are isolated from the patient's peripheral blood mononuclear cells (PBMCs) and an antigen to be presented is delivered to the isolated dendritic cells, through which the dendritic cells are activated and then injected back into the patient so that the antigen is delivered from the injected dendritic cells to T cells. In the latter, *ex vivo* dendritic cell culture method and antigen delivery method are important, and currently used antigen presentation methods include transfection of DNA of an antigen to be presented using virus or nucleofection, or antigen delivery targeting dendritic cells in which an antigen is bound to an antibody targeting the dendritic cells.

Currently, the biggest problems in dendritic cell vaccines are that severe chronic inflammatory phenomena in the body and the Warburg effect are exhibited, and it is considered very difficult to achieve effective activation of anticancer immune cells under the cancer microenvironment in which immunosuppressive cytokines, immunosuppressive T cells, dendritic cells, and the like are present.

### Technical Problem

An object of the present invention is to provide an Epstein-Barr virus (EBV)-positive cancer-specific tumor antigen epitope, and a composition for activating T cells which comprises the same.

Another object of the present invention is to provide an antigen-presenting cell loaded with the epitope of the present invention, the antigen-presenting cell being capable of activating T cells for cancer treatment.

Another object of the present invention is to provide a method for producing epitope-loaded antigen-presenting cells for cancer treatment.

Another object of the present invention is to provide a T cell, activated by the antigen-presenting cell loaded with the epitope of the present invention.

Still yet another object of the present invention is to provide a method for activating T cells for cancer treatment.

Still yet another object of the present invention is to provide a method for treating cancer using an epitope-loaded antigen-presenting cell.

Another object of the present invention is to provide a method for treating cancer using the T cells activated by the epitope-loaded antigen-presenting cells.

However, the technical problem to be solved by the present invention is not limited to the above-mentioned problems, and other problems which are not mentioned will be clearly understood by those skilled in the art from the following description.

### Solution to Problem

According to an embodiment of the present invention, there is provided a cancer-specific tumor antigen epitope.

In the present invention, the "cancer-specific tumor antigen epitope" is derived from a protein antigen which is present only in cancer cells and is not present in normal cells. In the present invention, the cancer-specific tumor antigen epitope includes at least one epitope recognized by T cell receptors; and such an epitope may preferably include epitopes present in Epstein-Barr virus (EBV)-positive cancer cells, including the EBV viral epitopes or cancer cell epitopes, and may more preferably be the Epstein-Barr virus (EBV)-positive cancer cell antigen which is Epstein-Barr virus latent membrane protein 2 (LMP2a), or Epstein-Barr nuclear antigen 1 (EBNA-1), or an epitope thereof.

In the present invention, the "Epstein-Barr virus latent membrane protein 2 (LMP2a)" is one of several EBV genes expressed in all of type II and III diseases/malignancies. LMP2a corresponds to a transmembrane protein that acts as a negative modulator of B cell-receptor signaling and promotes cell survival through sequestering of tyrosine kinases. HLA-A2-restricted peptides are epitope-specific cytotoxic T lymphocyte epitopes, which are detectable *ex vivo* in 60% to 75% of individuals, and are the most immunodominant LMP epitopes in latent diseases. The CLGGLLTMV peptide corresponds to a potential target for NPC and HL treatments, since the epitope is conserved in biopsies taken from NPC and HL patients and, along with other EBV latent epitopes, is immunologically weak.

In the present invention, the "Epstein-Barr nuclear antigen 1 (EBNA-1)" corresponds to a multifunctional, dimeric viral protein associated with Epstein-Barr virus. This corresponds to an EBV protein found only in all EBV-associated malignancies. This plays an important role in maintaining the altered state that cells take when the cells are infected with EBV EBNA-1, on the other hand, corresponds to a glycine-alanine repeat sequence that separates a protein into amino- and carboxy-terminal domains. This sequence serves to stabilize a protein, prevent proteasomal breakdown, and impair antigen processing and MHC class I-restricted antigen presentation. Thus, the EBNA-1 inhibits CD8-restricted cytotoxic T cell responses against virus-infected cells. The EBNA-1 is expressed by Qp promoter in all latency programs and corresponds to the only viral protein expressed in latency program I.

In the present invention, the "epitope" refers to an epitope that is not present in a reference such as normal, non-cancerous cells or germline cells and is found in cancer cells. For example, the epitope may be, but is not limited to, a 10-mer to 20-mer, and preferably a 15-mer.

In the present invention, the epitope may exhibit binding affinity with at least one of HLA-A, HLA-B, HLA-C, HLA-E, HLA-F, HLA-G, β2-microglobulin, HLA-DPA1, HLA-DPB1, HLA-DQA1, HLA-DQB1, HLA-DRA1, HLA-DRB1, HLA-DRB3, HLA-DRB4, HLA-DRB5, HLA-DM, HLA-DOA, and HLA-DOB loci so that T cells, preferably memory T cells, extracted from human blood can have efficacy. Among these loci, the epitope may include those exhibiting high binding affinity with at least one of the HLA types that are most expressed by Koreans, for example, HLA-A^{∗}2402, HLA-A^{∗}A0201, HLA-A^{∗}3303, HLA-A^{∗}1101, HLA-A^{∗}0206, HLA-A^{∗}3101, HLA-B^{∗}5101, HLA-B^{∗}4403, HLA-B^{∗}5401, HLA-B^{∗}5801, and HLA-B^{∗}3501.

Preferably, in the present invention, the epitope has high binding affinity for HLA-A^{∗}2402 and may include epitopes of LMP2a antigen, each being a peptide represented by any one of SEQ ID NOs: 1 to 3, SEQ ID NOs: 7 to 9, SEQ ID NOs: 11 to 15, SEQ ID NOs: 19 to 21, SEQ ID NO: 23 to 39, SEQ ID NO: 43 to 45, SEQ ID NO: 47 to 51, SEQ ID NO: 55 to 57, SEQ ID NO: 59 to 63, SEQ ID NO: 67 to 69, SEQ ID NO: 71 to 75, SEQ ID NO: 79 to 81, SEQ ID NO: 83 to 111, SEQ ID NOs: 115 to 117, SEQ ID NOs: 119 to 122; or the epitope has high binding affinity for HLA-A^{∗}3101 and may include epitopes of EBNA-1 antigen, each being a peptide represented by any one of SEQ ID NOs: 124 to 127, SEQ ID NOs: 129 to 149, SEQ ID NOs: 151 to 154, SEQ ID NOs: 156 to 158, SEQ ID NOs: 160 to 163, SEQ ID NOs: 165 to 167, SEQ ID NOs: 168 to 176, SEQ ID NOs: 178 to 181, SEQ ID NOs: 183 to 194, and SEQ ID NOs: 196 to 199.

Here, in the present invention, for a method of measuring the epitope-HLA affinity, NetMHC 3.4 (URL: www.cbs.dtu.dk/services/NetMHC-3.4/) may be used to predict whether an epitope binds to a specific HLA allele. However, the present invention is not limited thereto.

In the present invention, the "HLA" or "human leukocyte antigen" refers to human gene that encodes a major histocompatibility complex (MHC) protein on the surface of cells that are responsible for regulation of the immune system. "HLA-I" or "HLA class I" refers to human MHC class I gene including HLA-A, HLA-B, HLA-C, HLA-E, HLA-F, HLA-G, and β2-microglobulin loci. "HLA-II" or "HLA class II" refers to human MHC class II gene including HLA-DPA1, HLA-DPB1, HLA-DQA1, HLA-DQB1, HLA-DRA1, HLA-DRB1, HLA-DRB3, HLA-DRB4, HLA-DRB5, HLA-DM, HLA-DOA, and HLA-DOB loci.

In the present invention, the cancer may be Epstein-Barr virus (EBV)-positive cancer, including EBV-positive gastric cancer, EBV-positive cervical cancer, EBV-positive Burkitt's lymphoma, EBV-positive T cell lymphoma, EBV-positive breast cancer, EBV-positive leiomyosarcoma, EBV-positive smooth muscle tumor, EBV-positive Hodgkin lymphoma, EBV-positive nasopharyngeal cancer, or EBV-positive post-transplant lymphoproliferative disorder (PTLD), with EBV-positive gastric cancer being preferred.

According to another embodiment of the present invention, there is provided a nucleic acid molecule, encoding a cancer-specific tumor antigen epitope provided in the present invention, preferably an epitope of LMP2a or EBNA-1 which is an antigen of Epstein-Barr virus (EBV)-positive cancer cells.

The nucleic acid molecule of the present invention includes any nucleic acid molecule obtained by converting an amino acid sequence of a polypeptide provided in the present invention into a polynucleotide sequence as known to those skilled in the art. Thus, various polynucleotide sequences may be prepared due to open reading frame (ORF), all of which are also included in the nucleic acid molecule of the present invention.

According to yet another embodiment of the present invention, there is provided an expression vector, into which the isolated nucleic acid molecule provided in the present invention is inserted.

In the present invention, the "vector" is a nucleic acid molecule which is capable of transporting another nucleic acid linked thereto. One type of vector is a "plasmid," which refers to circular double-stranded DNA into which an additional DNA segment can be ligated. Another type of vector is a phage vector. Yet another type of vector is a viral vector, where an additional DNA segment can be ligated into the viral genome. Certain vectors are capable of autonomous replication in a host cell into which they are introduced (for example, bacterial vectors having a bacterial origin of replication and episomal mammalian vectors). Other vectors (for example, non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, "plasmid" and "vector" may be used interchangeably as the plasmid is the most commonly used form among vectors.

Specific examples of the expression vector in the present invention may be selected from, but are not limited to, the group consisting of commercially widely used pCDNA vectors, F, R1, RP1, Col, pBR322, ToL, Ti vectors; cosmids; phages such as lambda, lambdoid, M13, Mu, p1 P22, Qµµ, T-even, T2, T3, T7; plant viruses. Any expression vector known, to those skilled in the art, as expression vectors can be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation. However, the present invention is not limited thereto, and those skilled in the art may adopt and use an introduction method appropriate for the expression vector and the host cell which are used. The vector may preferably contain at least one selection marker. However, the present invention is not limited thereto, and selection can be made using the vector that contains no selection marker, depending on whether or not a product is produced. The selection marker is selected depending on the target host cell, which is done using methods already known to those skilled in the art, and thus the present invention has no limitation thereon.

In order to facilitate purification of the nucleic acid molecule of the present invention, a tag sequence may be inserted into and fused to an expression vector. The tag includes, but is not limited to, hexa-histidine tag, hemagglutinin tag, myc tag, or flag tag, and any tag known to those skilled in the art which facilitates purification can be used in the present invention.

According to still yet another embodiment of the present invention, there is provided a host cell, transfected with the expression vector provided in the present invention.

In the present invention, the "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal, or cellular origin, and may be, for example, bacterial cells such as E. coli, Streptomyces, Salmonella typhimurium; fungal cells such as yeast cells and Pichia pastoris; insect cells such as Drosophila and Spodoptera Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells. However, the host cell is not limited thereto, and any cell known to those skilled in the art which can be used as a host cell is available.

According to still yet another embodiment of the present invention, there is provided a composition for activating T cells, comprising a cancer-specific tumor antigen epitope provided in the present invention, a nucleic acid molecule encoding the same, an expression vector into which the nucleic acid molecule is inserted, or a host cell transformed with the expression vector.

As used herein, "activation of T cells" refers to a population of monoclonal (for example, encoding the same TCR) or polyclonal (for example, having clones encoding different TCRs) T cells that have T cell receptors recognizing at least one tumor antigen peptide. Activated T cells may include one or more subtypes of T cells, including, but not limited to, one or more selected from the group consisting of cytotoxic T cells, helper T cells, natural killer T cells, γδ T cells, regulatory T cells, and memory T cells, with memory T cells being preferred.

In the present invention, the activated T cells can treat a cancerous or neoplastic condition or prevent recurrence, progression, or metastasis of cancer while avoiding the defense mechanism of cancer cells.

According to still yet another embodiment of the present invention, there is provided an antigen-presenting cell (APC) loaded with a cancer-specific tumor antigen epitope provided in the present invention.

In the present invention, the antigen-presenting cells may include at least one of dendritic cell (DC), B cell, and macrophage, with dendritic cell being preferred.

In the present invention, the antigen-presenting cells may be isolated from an individual.

In the present invention, the "dendritic cell" refers to any member of a diverse population of morphologically similar cell types found in lymphoid or non-lymphoid tissues. These cells are characterized by their distinctive morphology and high expression levels of surface class I and class II MHC molecules, which are proteins that present antigenic peptides to T cells. DCs, other APCs, and T cells may be isolated or derived (such as differentiated) from a number of tissue sources, and conveniently from peripheral blood, such as peripheral blood mononuclear cells (PBMCs) derived from peripheral blood.

In the present invention, the antigen-presenting cell can induce differentiation and proliferation of cancer antigen-specific T cells, preferably memory T cells, thereby treating a cancerous or neoplastic condition or preventing recurrence, progression, or metastasis of cancer while avoiding the defense mechanism of cancer cells.

According to still yet another embodiment of the present invention, there is provided a fusion protein, comprising: a cancer-specific tumor antigen epitope provided in the present invention; and a dendritic cell-specific antibody or a fragment thereof.

The fusion protein provided in the present invention enables the cancer-specific tumor antigen epitope provided in the present invention to be loaded on dendritic cells.

In the present invention, the dendritic cell-specific antibody may include, but is not limited to, antibodies specific for DCIR on dendritic cells, MHC class I, MHC class II, CD1, CD2, CD3, CD4, CD8, CD11b, CD14, CD15, CD16, CD19, CD20, CD29, CD31, CD40, CD43, CD44, CD45, CD54, CD56, CD57, CD58, CD83, CD86, CMRF-44, CMRF-56, DCIR, DC-ASPGR, CLEC-6, CD40, BDCA-2, MARCO, DEC-205, Clec9A, 33D1, mannose receptor, Langerin, DECTIN-1, B7-1, B7-2, IFN-γ receptor, IL-2 receptor, ICAM-1, Fcγ receptor, LOX-1, or ASPGR.

The cancer-specific tumor antigen epitope in the fusion protein of the present invention may be conjugated to the dendritic cell-specific antibody or a fragment thereof. Here, the term "conjugate" refers to any material formed by joining two parts together. A representative conjugate according to the present invention includes those formed by joining an antigen together with an antibody and a TLR agonist. The term "conjugation" refers to a process of forming a conjugate and generally indicates physical coupling, for example, covalent bond, cocoordinate covalent bond, or second binding force, for example, Van der Waals binding force. The process of linking the antigen to the antibody may also be done via a non-covalent association such as a dockerin-cohesin association (as described in U.S. Patent Publication No. 20100135994, Banchereau et al. relevant portions incorporated herein by reference) or via a direct chemical linkage by forming a peptide or chemical bond.

According to another embodiment of the present invention, there is provided a method for producing an antigen-presenting cell, in which the antigen-presenting cell is loaded with a cancer-specific tumor antigen epitope provided in the present invention.

In the present invention, the antigen-presenting cell may include one or more of dendritic cell, B cell, and macrophage, with dendritic cell being preferred.

In the present invention, the dendritic cells (such as immature dendritic cells) may be obtained from a variety of sources including autologous sources, that is, derived from a target individual. The dendritic cells may preferably be obtained from peripheral blood mononuclear cells (PBMCs) derived from peripheral blood, and more preferably be obtained by isolating monocytes from individual-derived PBMCs and contacting the monocytes with a plurality of cytokines. Here, the type of cytokine that induces differentiation of the monocytes into dendritic cells is not particularly limited, and may include, for example, one or more of GM-CSF and IL-4.

In the present invention, the "target individual" means an individual who has or is at high risk of developing cancer.

In the present invention, once antigen-presenting cells are prepared as described above, the antigen-presenting cells may be loaded with a cancer-specific tumor antigen epitope of the present invention. In general, immature dendritic cells capture an antigen through phagocytosis or receptor-mediated endocytosis, process the antigen through a series of intracellular processes and then cause an antigenic peptide to be loaded on MHC and presented to T lymphocytes. With the process of processing an antigen, the dendritic cells become more mature, which makes them lose receptors used for phagocytosis and endocytosis, exhibit increased expression of MHC class I, II, costimulatory molecules, and adhesion molecules, and express new chemokine receptors. This allows the dendritic cells to migrate to T lymphocyterich areas of the surrounding lymph nodes, and to present the antigen to T lymphocytes, thereby causing a T lymphocyte immune response.

In an example of the present invention, in order for the cancer-specific tumor antigen epitope to be loaded on the antigen-presenting cell, the antigen-presenting cell may be contacted with the cancer-specific tumor antigen epitope of the present invention, and preferably, a step of pulsing, with the cancer-specific tumor antigen epitope of the present invention, the antigen-presenting cells, for example, immature dendritic cells, or antigen-presenting cells (such as dendritic cells) contained in or derived (for example, differentiated) from PBMCs may be performed. As known in the art, pulsing refers to a process of mixing cells, such as dendritic cells, with a solution containing a cancer-specific tumor antigen epitope peptide of the present invention, and then optionally removing the cancer-specific tumor antigen epitope peptide from the mixture. In the present invention, when the immature dendritic cells are contacted with the cancer-specific tumor antigen epitope, treatment with tolllike receptor agonists may be performed to further induce maturation of a population of immature dendritic cells. Here, exemplary TLR agonists include, but are not limited to, polyIC, MALP, and R848.

In another example of the present invention, in order for the cancer-specific tumor antigen epitope to be loaded on the antigen-presenting cell, it is possible to perform nucleofection of the antigen-presenting cell with an expression vector, preferably a plasmid, into which a nucleic acid molecule encoding the cancer-specific tumor antigen epitope is inserted. Here, the nucleofection may be performed by any useful means in the art, including, for example, Amaxa^{®} nucleofection system or InVitrogen^{®} nucleofection system.

In yet another example of the present invention, in order for the cancer-specific tumor antigen epitope to be loaded on the antigen-presenting cell, such loading may be performed using a fusion protein that contains the cancer-specific tumor antigen epitope provided in the present invention; and a dendritic cell-specific antibody or a fragment thereof.

According to still yet another embodiment of the present invention, there is provided a T cell activated by an antigen-presenting cell provided in the present invention.

In the present invention, the T cells may be isolated from an individual.

In the present invention, the T cells refer to a population of monoclonal (for example, encoding the same TCR) or polyclonal (for example, having clones encoding different TCRs) T cells that have T cell receptors recognizing at least one tumor antigen peptide, and may include one or more subtypes of T cells, including, but not limited to, one or more selected from the group consisting of cytotoxic T cells, helper T cells, natural killer T cells, γδ T cells, regulatory T cells, and memory T cells, with memory T cells being preferred.

In the present invention, the "memory T cells" are T cells that have previously encountered and responded to their specific antigen, or T cells that have differentiated from activated T cells. Although tumor-specific memory T cells make up a small portion of the total T cell amount, they play an important function in surveillance of tumor cells during a person's entire lifespan. In a case where tumor-specific memory T cells encounter tumor cells that express their specific tumor antigen, the memory T cells are immediately activated and clonally expanded. The activated and expanded T cells differentiate into effector T cells to kill tumor cells with high efficiency. Memory T cells are important for establishing and maintaining long-term tumor antigen-specific responses of T cells. In the present invention, activated T cells, preferably activated memory T cells, specifically recognize antigens on cancer cells, so that such T cells can treat a cancerous or neoplastic condition or prevent recurrence, progression, or metastasis of cancer while avoiding the defense mechanism of cancer cells.

According to still yet another embodiment of the present invention, there is provided a method for activating T cells using an antigen-presenting cell (APC) provided in the present invention.

In the present invention, for activation of the T cells, the T cells may be co-cultured with antigen-presenting cells loaded with a cancer-specific tumor antigen epitope of the present invention.

In the present invention, the T cells may be obtained from various sources including autologous sources, that is, derived from a target individual, may preferably be obtained from peripheral blood mononuclear cells (PBMCs) derived from peripheral blood, and may more preferably be obtained from non-adherent portions of the peripheral blood mononuclear cells. In an example of the present invention, the non-adherent portions of the PBMCs may be obtained by density gradient centrifugation of a peripheral blood sample, or may be obtained by performing culture with at least one cytokine (such as IL-2) in the presence or absence of an anti-CD3 antibody (such as OKT3).

In the present invention, the T cells refer to a population of monoclonal (for example, encoding the same TCR) or polyclonal (for example, having clones encoding different TCRs) T cells that have T cell receptors recognizing a tumor antigen peptide, and may include one or more subtypes of T cells, including, but not limited to, one or more selected from the group consisting of cytotoxic T cells, helper T cells, natural killer T cells, γδ T cells, regulatory T cells, and memory T cells, with memory T cells being preferred.

In addition, in the present invention, the T cells and the antigen-presenting cells may be derived from the same individual, such as an individual suffering from cancer, preferably EBV-positive cancer (for example, low to medium grade cancer). However, the present invention is not limited thereto.

In the present invention, for activation of the T cells, the T cells may be co-cultured with antigen-presenting cells of the present invention for any one or more time periods of 1, 2, 3, 4, 6, 8, 10, 12, 14, 16, 18, 20, 22, 24, 26, 28, or 30 days, and preferably for 1 to 21 days, 1 to 14 days, 2 to 10 days, 2 to 5 days, 2 to 5 days, 3 days, 5 days, 7 days, 10 days, 14 days, 16 days, 18 days, or 21 days. However, the present invention is not limited thereto.

In the present invention, during the co-culture of the T cells with antigen-presenting cells of the present invention, one or more cytokines may be added to prime the T cells so that activation, maturation and/or proliferation of the T cells are promoted and the T cells subsequently differentiate into memory T cells. Exemplary cytokines that may be used at this stage include, but are not limited to, interleukin-2 (IL-2), interleukin-4 (IL-4), interleukin-7 (IL-7), interleukin-15 (IL-15), interleukin-21 (IL-21), or combinations thereof, and the like.

In addition, in the present invention, during the co-culture of the T cells with antigen-presenting cells of the present invention, a fusion protein comprising a cytokine and an immunoglobulin heavy chain constant region may be added to prime the T cells so that activation, maturation and/or proliferation of the T cells are promoted and the T cells subsequently differentiate into memory T cells. Here, the cytokine may include, but is not limited to, interferon-γ (IFN-γ), interleukin-2 (IL-2), interleukin-4 (IL-4), interleukin-12 (IL-12), IL-18, and tumor necrosis factor (TNF), or granulocyte macrophage colony stimulating factor (GMCSF). The immunoglobulin heavy chain constant region may also be, but is not limited to, an immunoglobulin hinge region, and an immunoglobulin heavy chain constant region optionally selected from the group consisting of CH2 domain, CH3 domain, and CH4 domain, or combinations thereof. In addition, the immunoglobulin heavy chain constant region may be derived from immunoglobulins belonging to any of five immunoglobulin classes called in the art as IgA (Igα), IgD (Igδ), IgE (Igε), IgG (Igγ), and IgM (Igµ), and may preferably be an immunoglobulin heavy chain constant region derived from the IgG class.

In addition, in the present invention, during the co-culture of the T cells with antigen-presenting cells of the present invention, a fusion protein that contains ligand binding to a cell surface protein which is highly expressed in memory T cells; and an immunoglobulin heavy chain constant region, may be added to prime the T cells so that activation, maturation and/or proliferation of the T cells are promoted and the T cells subsequently differentiate into memory T cells. Here, the cell surface protein which is highly expressed in memory T cells may be CD27, CXCR3, or CD62L. The ligand capable of binding to CD27 may be CD70; the ligand capable of binding to CXCR3 may be CXCR9 or CXCR10; and the ligand capable of binding to CD62L may be GlyCAM-1, CD34, MadCAM-1, or PSGL-1. However, the present invention is not limited thereto. In addition, the immunoglobulin heavy chain constant region may be derived from immunoglobulins belonging to any of five immunoglobulin classes called in the art as IgA (Igα), IgD (Igδ), IgE (Igε), IgG (Igγ), and IgM (Igµ), and may preferably be an immunoglobulin heavy chain constant region derived from the IgG class.

According to still yet another embodiment of the present invention, there is provided an immunotherapeutic agent, comprising, as an active ingredient, an antigen-presenting cell loaded with a cancer-specific tumor antigen epitope provided in the present invention. The immunotherapeutic agent according to the present invention can increase immune responses or may selectively increase some of immune responses desired for treatment or prevention of a certain disease, for example, cancer.

According to still yet another embodiment of the present invention, there is provided an anticancer vaccine or a pharmaceutical composition for preventing or treating cancer, comprising, as an active ingredient, an antigen-presenting cell loaded with a cancer-specific tumor antigen epitope provided in the present invention; and/or an activated T cell.

As used herein, the term "cancer" refers to or indicates a physiological condition characterized by cell growth in mammals which is not regulated in a typical manner. The cancer to be prevented, ameliorated, or treated in the present invention may be Epstein-Barr virus (EBV)-positive cancer, including, but not limited to, EBV-positive gastric cancer, EBV-positive cervical cancer, EBV-positive Burkitt's lymphoma, EBV-positive T cell lymphoma, EBV-positive breast cancer, EBV-positive leiomyosarcoma, EBV-positive smooth muscle tumor, EBV-positive Hodgkin lymphoma, EBV-positive nasopharyngeal cancer, or EBV-positive post-transplant lymphoproliferative disorder (PTLD), with EBV-positive gastric cancer being preferred.

The antigen-presenting cell provided in the present invention enables induction of differentiation and proliferation of EBV-positive cancer antigen-specific T cells, preferably memory T cells, and the memory T cells thus activated can treat a cancerous or neoplastic condition or prevent recurrence, progression, or metastasis of cancer while avoiding the defense mechanism of cancer cells.

The anticancer vaccine according to the present invention may involve both an immunization method performed by single administration and an immunization method performed by continuous administration.

In the present invention, the "prevention" may include, without limitation, any act of blocking symptoms of cancer, or suppressing or delaying the symptoms, using the pharmaceutical composition of the present invention.

In addition, in the present invention, the "treatment" may include, without limitation, any act of ameliorating or beneficially altering symptoms of cancer, using the pharmaceutical composition of the present invention.

In the present invention, the pharmaceutical composition may be characterized by being in the form of capsules, tablets, granules, injections, ointments, powders, or beverages, and the pharmaceutical composition may be characterized by being targeted to humans.

In the present invention, the pharmaceutical composition may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition of the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

As used herein, the term "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, time of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the present invention in any way. The pharmaceutical composition according to the present invention may be formulated in the form of pills, sugar-coated tablets, capsules, liquids, gels, syrups, slurries, or suspensions.

According to still yet another embodiment of the present invention, there is provided a method for preventing or treating cancer, comprising a step of administering, to a target individual, an antigen-presenting cell loaded with a cancer-specific tumor antigen epitope provided in the present invention; and/or an activated T cell.

In the present invention, the cancer may be Epstein-Barr virus (EBV)-positive cancer, including, but not limited to, EBV-positive gastric cancer, EBV-positive cervical cancer, EBV-positive Burkitt's lymphoma, EBV-positive T cell lymphoma, EBV-positive breast cancer, EBV-positive leiomyosarcoma, EBV-positive smooth muscle tumor, EBV-positive Hodgkin lymphoma, EBV-positive nasopharyngeal cancer, or EBV-positive post-transplant lymphoproliferative disorder (PTLD), with EBV-positive gastric cancer being preferred.

Dose, schedule, and route of administration of the antigen-presenting cell loaded with a cancer-specific tumor antigen epitope provided in the present invention or the activated T cell may be determined depending on the size and condition of an individual, and in accordance with standard pharmaceutical practice. Exemplary routes of administration include intravenous, intraarterial, intraperitoneal, intrapulmonary, intravascular, intramuscular, intratracheal, subcutaneous, intraocular, intrathecal, or transdermal route.

A dose of cells administered to an individual may vary depending, for example, on the particular type of cells being administered, the route of administration, and the particular type and stage of cancer being treated. The amount should be sufficient to produce a desirable response, such as a therapeutic response against cancer, but without severe toxicity or adverse events. In some embodiments, the amount of activated T cells or antigen-presenting cells (such as dendritic cells) to be administered is a therapeutically effective amount. In some embodiments, the amount of cells (such as dendritic cells loaded with a cancer-specific tumor antigen epitope or activated T cells) is an amount sufficient to decrease the size of a tumor, decrease the number of cancer cells, or decrease the growth rate of a tumor by any one of at least about 10%, 20%, 30%, 40%, 50%, 60%, 70%, 80%, 90%, 95%, or 100%, as compared with the corresponding tumor size, number of cancer cells, or tumor growth rate in the same individual prior to treatment or as compared with the corresponding activity in other individuals having not received the treatment. The magnitude of effects may be measured using standard methods, such as *in vitro* assays with purified enzymes, cell-based assays, animal models, or experiments using humans.

In an embodiment of the present invention, the antigen-presenting cells (such as dendritic cells) loaded with a cancer-specific tumor antigen epitope of the present invention may be administrated at a dose of any of 1×10⁵ to 5×10⁵, 5×10⁵ to 1×10⁶, 1×10⁶ to 2×10⁶, 2×10⁶ to 3×10⁶, 3×10⁶ to 4×10⁶, 4×10⁶ to 5×10⁶, 5×10⁶ to 6×10⁶, 6×10⁶ to 7×10⁶, 7×10⁶ to 8×10⁶, 8×10⁶ to 1×10⁸, 1×10⁶ to 3×10⁶, 3×10⁶ to 5×10⁶, 5×10⁶ to 7×10⁶, 2×10⁶ to 4×10⁶, 1×10⁶ to 5×10⁶, or 5×10⁶ to 1×10⁷ cells/individual. However, the present invention is not limited thereto.

In another embodiment of the present invention, the antigen-presenting cells (e.g., dendritic cells) loaded with a cancer-specific tumor antigen epitope of the present invention may be administrated at a dose of any of 1×10⁴ to 5×10⁴, 5×10⁴ to 1×10⁵, 1×10⁵ to 2×10⁵, 2×10⁵ to 4×10⁵, 4×10⁵ to 6×10⁵, 6×10⁵ to 8×10⁵, 8×10⁵ to 1×10⁶, 1×10⁶ to 2×10⁶, 2×10⁶ to 1×10⁷, 1×10⁴ to 1×10⁵, 1×10⁵ to 1×10⁶, 1×10⁶ to 1×10⁷, 1×10⁴ to 1×10⁶, or 1×10⁵ to 1×10⁷ cells/kg. However, the present invention is not limited thereto.

In addition, in an embodiment of the present invention, the activated T cells of the present invention may be administrated at a dose of any of 1×10⁸ to 5×10⁸, 5×10⁸ to 9×10⁸, 9×10⁸ to 1×10⁹, 1×10⁹ to 2×10⁹, 2×10⁹ to 3×10⁹, 3×10⁹ to 4×10⁹, 4×10⁹ to 5×10⁹, 5×10⁹ to 6×10⁹, 6×10⁹ to 1×10¹⁰, 1×10⁹ to 3×10⁹, 3×10⁹ to 5×10⁹, 5×10⁹ to 7×10⁹, 7×10⁹ to 1×10¹⁰, 1×10⁹ to 5×10⁹, 5×10⁹ to 1×10¹⁰, 3×10⁹ to 7×10⁹, 1×10¹⁰ to 1.5×10¹⁰, 1×10¹⁰ to 2×10¹⁰, or 1×10⁹ to 1×10¹⁰ cells/individual. However, the present invention is not limited thereto.

In another embodiment of the present invention, the activated T cells of the present invention may be administrated at a dose of any of 1×10⁷ to 1 ×10⁸, 1×10⁸ to 2×10⁸, 2×10⁸ to 4×10⁸, 4×10⁸ to 6×10⁸, 6×10⁸ to 8×10⁸, 8×10⁸ to 1×10⁹, 1×10⁹ to 2×10⁹, 2×10⁹ to 4×10⁹, 4×10⁹ to 1×10¹⁰, 2×10⁸ to 6×10⁸, 6×10⁸ to 1×10⁹, 1×10⁸ to 2×10⁸, 2×10⁸ to 2×10⁹, 1×10⁷ to 1×10⁸, 1×10⁸ to 1×10⁹, 1×10⁹ to 1×10¹⁰, or 1×10⁷ to 1×10⁹ cells/kg. However, the present invention is not limited thereto.

In the present invention, a stabilizer or excipient such as human albumin may be used together with administration of the antigen-presenting cells (such as dendritic cells) loaded with a cancer-specific tumor antigen epitope and/or the activated T cells.

In the present invention, dose and dosing schedule of the antigen-presenting cells (such as dendritic cells) loaded with a cancer-specific tumor antigen epitope and/or the activated T cells may be adjusted over the course of treatment based on the judgment of the administering physician. In some embodiments, the activated T cells may be administered at any time point of about 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, or 1 month after the antigen-presenting cells loaded with a tumor antigen peptide are administered, or may be administered simultaneously with the antigen-presenting cells. However, the present invention is not limited thereto.

In the present invention, administration of the antigen-presenting cells (such as dendritic cells) loaded with a tumor specific epitope and/or the activated T cell may be done alone or in combination with other therapies, such as surgery, radiation therapy, gene therapy, immunotherapy, bone marrow transplantation, stem cell transplantation, hormone therapy, targeted therapy, cryotherapy, ultrasound therapy, photodynamic therapy, chemotherapy, or the like. Additionally, a person having a greater risk of developing a proliferative disease may receive treatments to inhibit and/or delay development of the disease.

### Advantageous Effects of Invention

The antigen-presenting cell, that is, dendritic cell, loaded with an Epstein-Barr virus (EBV)-positive cancer-specific tumor antigen epitope provided in the present invention enables rapid and effective induction of differentiation and proliferation of the cancer antigen-specific T cells, preferably memory T cells, and the memory T cells thus activated can treat an Epstein-Barr virus (EBV)-positive cancerous or neoplastic condition or prevent recurrence, progression, or metastasis of cancer while avoiding the defense mechanism of cancer cells.

In the conventional adoptive T cell therapies, it takes a long time of 3 to 6 months to produce a large number of T cells for treatment of cancer patients, which poses a big problem in the cell production process in immune cell therapy. However, according to the present invention, 10⁹ autologous memory T cells, which should be used for patient treatment, can be produced within three weeks, and cost reduction and minimized infection risk factors to external contaminants can be achieved. Accordingly, according to the present invention, there is provided a technique that can be applied to terminal cancer patients because such a technique makes rapid therapeutic approaches available for a larger number of solid cancer patients.

### Brief Description of Drawings

FIGS. 1 to 4 illustrate results obtained by performing ELISPOT on each of LMP2a-derived epitopes in order to check the degree of T cell activation caused by the epitope in normal human-derived PBMCs, according to an embodiment of the present invention.
FIGS. 5 to 8 illustrate results obtained by performing ELISPOT on each of EBNA-1-derived epitopes in order to check the degree of T cell activation caused by the epitope in normal human-derived PBMCs, according to an embodiment of the present invention.

### Detailed Description of Invention

According to an embodiment of the present invention, there is provided an Epstein-Barr virus (EBV)-positive cancer-specific tumor antigen epitope, represented by any one of SEQ ID NOs: 1 to 3, SEQ ID NOs: 7 to 9, SEQ ID NOs: 11 to 15, SEQ ID NOs: 19 to 21, SEQ ID NOs: 23 to 39, SEQ ID NOs: 43 to 45, SEQ ID NOs: 47 to 51, SEQ ID NOs: 55 to 57, SEQ ID NOs: 59 to 63, SEQ ID NOs: 67 to 69, SEQ ID NOs: 71 to 75, SEQ ID NOs: 79 to 81, SEQ ID NOs: 83 to 111, SEQ ID NOs: 115 to 117, SEQ ID NOs: 119 to 122, SEQ ID NOs: 124 to 127, SEQ ID NOs: 129 to 149, SEQ ID NOs: 151 to 154, SEQ ID NOs: 156 to 158, SEQ ID NOs: 160 to 163, SEQ ID NOs: 165 to 167, SEQ ID NOs: 168 to 176, SEQ ID NOs: 178 to 181, SEQ ID Nos: 183 to 194, and SEQ ID NOs: 196 to 199.

According to another embodiment of the present invention, there is provided an antigen-presenting cell (APC) loaded with a cancer-specific tumor antigen epitope provided in the present invention.

According to yet another embodiment of the present invention, there is provided a T cell activated by an antigen-presenting cell provided in the present invention.

According to still yet another embodiment of the present invention, there is provided an anticancer vaccine or a pharmaceutical composition for preventing or treating cancer, comprising, as an active ingredient, an antigen-presenting cell loaded with a cancer-specific tumor antigen epitope provided in the present invention; and/or an activated T cell.

According to still yet another embodiment of the present invention, there is provided a method for preventing or treating cancer, comprising administering, to an individual, an effective amount of an antigen-presenting cell loaded with a cancer-specific tumor antigen epitope provided in the present invention; and/or an activated T cell.

Hereinafter, the present invention will be described in more detail by way of examples. These examples are only for describing the present invention in more detail, and it will be apparent to those skilled in the art that according to the gist of the present invention, the scope of the present invention is not limited by these examples.

### Examples

### [Example 1] Method for producing EBV-positive gastric cancer cell antigen epitopes

Prediction of epitopes was made such that the epitope has 15-mer amino acids in the entire sequence of LMP2a or EBNA-1 as shown in Tables 1 and 2; and the epitopes in the sequence listing, which are set forth in Tables 1 (LNP2a) and 2 (EBNA-1), were produced according to a conventional method related to peptide production processes.

**[Table 1]**

| SEQ ID NO | Amino acid sequence |
|---|---|
| SEQ ID NO: 1 | MGSLEMVPMGAGPPS |
| SEQ ID NO: 2 | EMVPMGAGPPSPGGD |
| SEQ ID NO: 3 | MGAGPPSPGGDPDGD |
| SEQ ID NO: 4 | PPSPGGDPDGDDGGN |
| SEQ ID NO: 5 | GGDPDGDDGGNNSQY |
| SEQ ID NO: 6 | DGDDGGNNSQYPSAS |
| SEQ ID NO: 7 | GGNNSQYPSASGSYG |
| SEQ ID NO: 8 | SQYPSASGSYGNTPT |
| SEQ ID NO: 9 | SASGSYGNTPTPPND |
| SEQ ID NO: 10 | SYGNTPTPPNDEERE |
| SEQ ID NO: 11 | TPTPPNDEERESNEE |
| SEQ ID NO: 12 | PNDEERESNEEPPPP |
| SEQ ID NO: 13 | ERESNEEPPPPYEDL |
| SEQ ID NO: 14 | NEEPPPPYEDLDWGN |
| SEQ ID NO: 15 | PPPYEDLDWGNGDRH |
| SEQ ID NO: 16 | EDLDWGNGDRHSDYQ |
| SEQ ID NO: 17 | WGNGDRHSDYQPLGN |
| SEQ ID NO: 18 | DRHSDYQPLGNQDPS |
| SEQ ID NO: 19 | DYQPLGNQDPSLYLG |
| SEQ ID NO: 20 | LGNQDPSLYLGLQHD |
| SEQ ID NO: 21 | DPSLYLGLQHDGNDG |
| SEQ ID NO: 22 | YLGLQHDGNDGLPPP |
| SEQ ID NO: 23 | QHDGNDGLPPPPYSP |
| SEQ ID NO: 24 | NDGLPPPPYSPRDDS |
| SEQ ID NO: 25 | PPPPYSPRDDSSQHI |
| SEQ ID NO: 26 | YSPRDDSSQHIYEEA |
| SEQ ID NO: 27 | DDSSQHIYEEAGRGS |
| SEQ ID NO: 28 | QHIYEEAGRGSMNPV |
| SEQ ID NO: 29 | EEAGRGSMNPVCLPV |
| SEQ ID NO: 30 | RGSMNPVCLPVIVAP |
| SEQ ID NO: 31 | NPVCLPVIVAPYLFW |
| SEQ ID NO: 32 | LPVIVAPYLFWLAAI |
| SEQ ID NO: 33 | VAPYLFWLAAIAASC |
| SEQ ID NO: 34 | LFWLAAIAASCFTAS |
| SEQ ID NO: 35 | AAIAASCFTASVSTV |
| SEQ ID NO: 36 | ASCFTASVSTVVTAT |
| SEQ ID NO: 37 | TASVSTVVTATGLAL |
| SEQ ID NO: 38 | STVVTATGLALSLLL |
| SEQ ID NO: 39 | TATGLALSLLLLAAV |
| SEQ ID NO: 40 | LALSLLLLAAVASSY |
| SEQ ID NO: 41 | LLLLAAVAS SYAAAQ |
| SEQ ID NO: 42 | AAVAS SYAAAQRKLL |
| SEQ ID NO: 43 | SSYAAAQRKLLTPVT |
| SEQ ID NO: 44 | AAQRKLLTPVTVLTA |
| SEQ ID NO: 45 | KLLTPVTVLTAVVTF |
| SEQ ID NO: 46 | PVTVLTAVVTFFAIC |
| SEQ ID NO: 47 | LTAVVTFFAICLTWR |
| SEQ ID NO: 48 | VTFFAICLTWRIEDP |
| SEQ ID NO: 49 | AICLTWRIEDPPFNS |
| SEQ ID NO: 50 | TWRIEDPPFNSLLFA |
| SEQ ID NO: 51 | EDPPFNSLLF ALL AA |
| SEQ ID NO: 52 | FNSLLFALLAAAGGL |
| SEQ ID NO: 53 | LFALLAAAGGLQGIY |
| SEQ ID NO: 54 | LAAAGGLQGIYVLVM |
| SEQ ID NO: 55 | GGLQGIYVLVMLVLL |
| SEQ ID NO: 56 | GIYVLVMLVLLILAY |
| SEQ ID NO: 57 | LVMLVLLILAYRRRW |
| SEQ ID NO: 58 | VLLILAYRRRWRRLT |
| SEQ ID NO: 59 | LAYRRRWRRLTVCGG |
| SEQ ID NO: 60 | RRWRRLTVCGGIMFL |
| SEQ ID NO: 61 | RLTVCGGIMFLACVL |
| SEQ ID NO: 62 | CGGIMFLACVLVLIV |
| SEQ ID NO: 63 | MFLACVLVLIVDAVL |
| SEQ ID NO: 64 | CVLVLIVDAVLQLSP |
| SEQ ID NO: 65 | LIVDAVLQLSPLLGA |
| SEQ ID NO: 66 | AVLQLSPLLGAVTVV |
| SEQ ID NO: 67 | LSPLLGAVTVVSMTL |
| SEQ ID NO: 68 | LGAVTVVSMTLLLLA |
| SEQ ID NO: 69 | TVVSMTLLLLAFVLW |
| SEQ ID NO: 70 | MTLLLLAFVLWLSSP |
| SEQ ID NO: 71 | LLAFVLWLSSPGGLG |
| SEQ ID NO: 72 | VLWLSSPGGLGTLGA |
| SEQ ID NO: 73 | S SPGGLGTLGAALLT |
| SEQ ID NO: 74 | GLGTLGAALLTLAAA |
| SEQ ID NO: 75 | LGAALLTLAAALALL |
| SEQ ID NO: 76 | LLTLAAALALLASLI |
| SEQ ID NO: 77 | AAALALLASLILGTL |
| SEQ ID NO: 78 | ALLASLILGTLNLTT |
| SEQ ID NO: 79 | SLILGTLNLTTMFLL |
| SEQ ID NO: 80 | GTLNLTTMFLLMLLW |
| SEQ ID NO: 81 | LTTMFLLMLLWTLVV |
| SEQ ID NO: 82 | FLLMLLWTLVVLLIC |
| SEQ ID NO: 83 | LLWTLVVLLICSSCS |
| SEQ ID NO: 84 | LVVLLICSSCSSCPL |
| SEQ ID NO: 85 | LICSSCSSCPLTKIL |
| SEQ ID NO: 86 | SCSSCPLTKILLARL |
| SEQ ID NO: 87 | CPLTKILLARLFLYA |
| SEQ ID NO: 88 | KILLARLFLYALALL |
| SEQ ID NO: 89 | ARLFLYALALLLLAS |
| SEQ ID NO: 90 | LYALALLLLASALIA |
| SEQ ID NO: 91 | ALLLLASALIAGGSI |
| SEQ ID NO: 92 | LASALIAGGSILQTN |
| SEQ ID NO: 93 | LIAGGSILQTNFKSL |
| SEQ ID NO: 94 | GSILQTNFKSLSSTE |
| SEQ ID NO: 95 | QTNFKSLSSTEFIPN |
| SEQ ID NO: 96 | KSLSSTEFIPNLFCM |
| SEQ ID NO: 97 | STEFIPNLFCMLLLI |
| SEQ ID NO: 98 | IPNLFCMLLLIVAGI |
| SEQ ID NO: 99 | FCMLLLIVAGILFIL |
| SEQ ID NO: 100 | LLIVAGILFILAILT |
| SEQ ID NO: 101 | AGILFILAILTEWGS |
| SEQ ID NO: 102 | FILAILTEWGSGNRT |
| SEQ ID NO: 103 | ILTEWGSGNRTYGPV |
| SEQ ID NO: 104 | WGSGNRTYGPVFMCL |
| SEQ ID NO: 105 | NRTYGPVFMCLGGLL |
| SEQ ID NO: 106 | GPVFMCLGGLLTMVA |
| SEQ ID NO: 107 | MCLGGLLTMVAGAVW |
| SEQ ID NO: 108 | GLLTMVAGAVWLTVM |
| SEQ ID NO: 109 | MVAGAVWLTVMTNTL |
| SEQ ID NO: 110 | AVWLTVMTNTLLSAW |
| SEQ ID NO: 111 | TVMTNTLLSAWILTA |
| SEQ ID NO: 112 | NTLLSAWILTAGFLI |
| SEQ ID NO: 113 | SAWILTAGFLIFLIG |
| SEQ ID NO: 114 | LTAGFLIFLIGFALF |
| SEQ ID NO: 115 | FLIFLIGFALF GVIR |
| SEQ ID NO: 116 | LIGFALF GVIRC CRY |
| SEQ ID NO: 117 | ALFGVIRCCRYCCYY |
| SEQ ID NO: 118 | VIRCCRYCCYYCLTL |
| SEQ ID NO: 119 | CRYCCYYCLTLESEE |
| SEQ ID NO: 120 | CYYCLTLESEERPPT |
| SEQ ID NO: 121 | LTLESEERPPTPYRN |
| SEQ ID NO: 122 | LESEERPPTPYRNTV |

**[Table 2]**

| SEQ ID NO | Amino acid sequence |
|---|---|
| SEQ ID NO: 123 | GGAGAGGGGRGRGGS |
| SEQ ID NO: 124 | AGGGGRGRGGSGGRG |
| SEQ ID NO: 125 | GRGRGGSGGRGRGGS |
| SEQ ID NO: 126 | GGSGGRGRGGSGGRG |
| SEQ ID NO: 127 | GRGRGGSGGRGRGGS |
| SEQ ID NO: 128 | GGSGGRGRGGSGGRR |
| SEQ ID NO: 129 | GRGRGGSGGRRGRGR |
| SEQ ID NO: 130 | GGSGGRRGRGRERAR |
| SEQ ID NO: 131 | GRRGRGRERARGGSR |
| SEQ ID NO: 132 | RGRERARGGSRERAR |
| SEQ ID NO: 133 | RARGGSRERARGRGR |
| SEQ ID NO: 134 | GSRERARGRGRGRGE |
| SEQ ID NO: 135 | RARGRGRGRGEKRPR |
| SEQ ID NO: 136 | RGRGRGEKRPRSPSS |
| SEQ ID NO: 137 | RGEKRPRSPSSQSSS |
| SEQ ID NO: 138 | RPRSPSSQSSSSGSP |
| SEQ ID NO: 139 | PSSQSSSSGSPPRRP |
| SEQ ID NO: 140 | SSSSGSPPRRPPPGR |
| SEQ ID NO: 141 | GSPPRRPPPGRRPFF |
| SEQ ID NO: 142 | RRPPPGRRPFFHPVG |
| SEQ ID NO: 143 | PGRRPFFHPVGDADY |
| SEQ ID NO: 144 | PFFHPVGDADYFEYL |
| SEQ ID NO: 145 | PVGDADYFEYLQEGG |
| SEQ ID NO: 146 | ADYFEYLQEGGPDGE |
| SEQ ID NO: 147 | EYLQEGGPDGEPDVP |
| SEQ ID NO: 148 | EGGPDGEPDVPPGAI |
| SEQ ID NO: 149 | DGEPDVPPGAIEQGP |
| SEQ ID NO: 150 | DVPPGAIEQGPTDDP |
| SEQ ID NO: 151 | GAIEQGPTDDPGEGP |
| SEQ ID NO: 152 | QGPTDDPGEGPSTGP |
| SEQ ID NO: 153 | DDPGEGPSTGPRGQG |
| SEQ ID NO: 154 | EGPSTGPRGQGDGGR |
| SEQ ID NO: 155 | TGPRGQGDGGRRKKG |
| SEQ ID NO: 156 | GQGDGGRRKKGGWFG |
| SEQ ID NO: 157 | GGRRKKGGWFGKHRG |
| SEQ ID NO: 158 | KKGGWFGKHRGQGGS |
| SEQ ID NO: 159 | WFGKHRGQGGSNPKF |
| SEQ ID NO: 160 | HRGQGGSNPKFENIA |
| SEQ ID NO: 161 | GGSNPKFENIAEGLR |
| SEQ ID NO: 162 | PKFENIAEGLRVLLA |
| SEQ ID NO: 163 | NIAEGLRVLLARSHV |
| SEQ ID NO: 164 | GLRVLLARSHVERTT |
| SEQ ID NO: 165 | LLARSHVERTTEEGN |
| SEQ ID NO: 166 | SHVERTTEEGNWVAG |
| SEQ ID NO: 167 | RTTEEGNWVAGVFVY |
| SEQ ID NO: 168 | EGNWVAGVFVYGGSK |
| SEQ ID NO: 169 | VAGVFVYGGSKTSLY |
| SEQ ID NO: 170 | FVYGGSKTSLYNLRR |
| SEQ ID NO: 171 | GSKTSLYNLRRGIAL |
| SEQ ID NO: 172 | SLYNLRRGIALAVPQ |
| SEQ ID NO: 173 | LRRGIALAVPQCRIT |
| SEQ ID NO: 174 | IALAVPQCRITPLSR |
| SEQ ID NO: 175 | VPQCRITPLSRLPFG |
| SEQ ID NO: 176 | RITPLSRLPFGMAPG |
| SEQ ID NO: 177 | LSRLPFGMAPGPGPQ |
| SEQ ID NO: 178 | PFGMAPGPGPQPGPL |
| SEQ ID NO: 179 | APGPGPQPGPLRESI |
| SEQ ID NO: 180 | GPQPGPLRESIVCYF |
| SEQ ID NO: 181 | GPLRESIVCYFMVFL |
| SEQ ID NO: 182 | ESIVCYFMVFLQTHI |
| SEQ ID NO: 183 | CYFMVFLQTHIFAEV |
| SEQ ID NO: 184 | VFLQTHIFAEVLKDA |
| SEQ ID NO: 185 | THIFAEVLKDAIKDL |
| SEQ ID NO: 186 | AEVLKDAIKDLVMTK |
| SEQ ID NO: 187 | KDAIKDLVMTKPAPT |
| SEQ ID NO: 188 | KDLVMTKPAPTCNIK |
| SEQ ID NO: 189 | MTKPAPTCNIKVTVC |
| SEQ ID NO: 190 | APTCNIKVTVCSFDD |
| SEQ ID NO: 191 | NIKVTVCSFDDGVDL |
| SEQ ID NO: 192 | TVCSFDDGVDLPPWF |
| SEQ ID NO: 193 | FDDGVDLPPWFPPMV |
| SEQ ID NO: 194 | VDLPPWFPPMVEGAA |
| SEQ ID NO: 195 | PWFPPMVEGAAAEGD |
| SEQ ID NO: 196 | PMVEGAAAEGDDGDD |
| SEQ ID NO: 197 | GAAAEGDDGDDGDEG |
| SEQ ID NO: 198 | EGDDGDDGDEGGDGD |
| SEQ ID NO: 199 | GDDGDEGGDGDEGEE |
| SEQ ID NO: 200 | GDEGGDGDEGEEGQE |

### [Example 2] ELISPOT results for T cells activated by dendritic cells loaded with selected LMP2a epitope

PBMCs extracted from the blood of three healthy humans (N5, N9, or N15) were separated into monocytes and leukocytes through magnetic-activated cell sorting, and the monocytes were cultured for 4 days in a culture supplemented with cytokines GM-CSF and IL-4 to differentiate into dendritic cells. To the culture, in which the monocytes had differentiated into dendritic cells, was added each group containing 10 or 11 epitope peptides as set forth in Table 3 together with poly(I:C) so that the respective peptides were transferred to the dendritic cells. The culture was matured for 1 day. In addition, the leukocytes were cultured for 3 days in a culture supplemented with anti-CD3/CD28 antibodies and cytokine IL-2.

**[Table 3]**

| Group | SEQ ID NO |
|---|---|
| 1 | SEQ ID NO: 1, SEQ ID NO: 13, SEQ ID NO: 25, SEQ ID NO: 37, SEQ ID NO: 49, SEQ ID NO: 61, SEQ ID NO: 73, SEQ ID NO: 85, SEQ ID NO: 97, SEQ ID NO: 109, SEQ ID NO: 121 |
| 2 | SEQ ID NO: 2, SEQ ID NO: 14, SEQ ID NO: 26, SEQ ID NO: 38, SEQ ID NO: 50, SEQ ID NO: 62, SEQ ID NO: 74, SEQ ID NO: 86, SEQ ID NO: 98, SEQ ID NO: 110, SEQ ID NO: 122 |
| 3 | SEQ ID NO: 3, SEQ ID NO: 15, SEQ ID NO: 27, SEQ ID NO: 39, SEQ ID NO: 51, SEQ ID NO: 63, SEQ ID NO: 75, SEQ ID NO: 87, SEQ ID NO: 99, SEQ ID NO: 111 |
| 4 | SEQ ID NO: 4, SEQ ID NO: 16, SEQ ID NO: 28, SEQ ID NO: 40, SEQ ID NO: 52, SEQ ID NO: 64, SEQ ID NO: 76, SEQ ID NO: 88, SEQ ID NO: 100, SEQ ID NO: 112 |
| 5 | SEQ ID NO: 5, SEQ ID NO: 17, SEQ ID NO: 29, SEQ ID NO: 41, SEQ ID NO: 53, SEQ ID NO: 65, SEQ ID NO: 77, SEQ ID NO: 89, SEQ ID NO: 101, SEQ ID NO: 113 |
| 6 | SEQ ID NO: 6, SEQ ID NO: 18, SEQ ID NO: 30, SEQ ID NO: 42, SEQ ID NO: 54, SEQ ID NO: 66, SEQ ID NO: 78, SEQ ID NO: 90, SEQ ID NO: 102, SEQ ID NO: 114 |
| 7 | SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115 |
| 8 | SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116 |
| 9 | SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117 |
| 10 | SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118 |
| 11 | SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119 |
| 12 | SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120 |
| 13 | SEQ ID NO: 1 to SEQ ID NO: 12 |
| 14 | SEQ ID NO: 13 to SEQ ID NO: 24 |
| 15 | SEQ ID NO: 25 to SEQ ID NO: 36 |
| 16 | SEQ ID NO: 37 to SEQ ID NO: 48 |
| 17 | SEQ ID NO: 49 to SEQ ID NO: 60 |
| 18 | SEQ ID NO: 61 to SEQ ID NO: 72 |
| 19 | SEQ ID NO: 73 to SEQ ID NO: 84 |
| 20 | SEQ ID NO: 85 to SEQ ID NO: 96 |
| 21 | SEQ ID NO: 97 to SEQ ID NO: 108 |
| 22 | SEQ ID NO: 109 to SEQ ID NO: 120 |
| 23 | SEQ ID NO: 121 and SEQ ID NO: 122 |

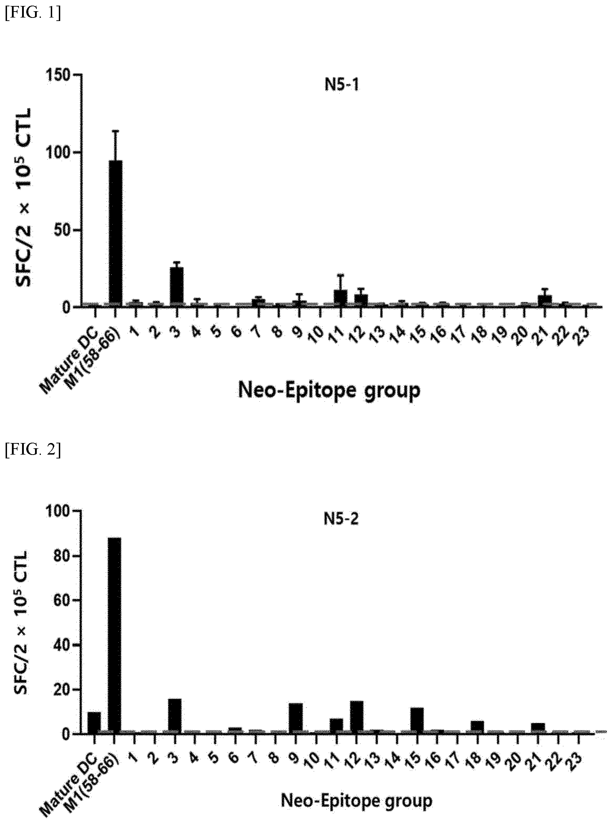

Then, CD8-expressing T cells were isolated from leukocytes, which were cultured in a culture supplemented with anti-CD3/CD28 antibody and cytokine IL-2, by magnetic-activated cell sorting, and co-cultured with mature dendritic cells at a ratio of 1:10 (dendritic cells:CD8-expressing T cells). In a case of the co-culture, the culture was mixed with a cytokine cocktail containing cytokines IL-4 and IL-7 that function to help survival and immune response of T cells, and the mixture was cultured. After 18 hours, secretion levels of IFN-γ in the T cells thus activated were measured with ELISPOT, and the results are illustrated in FIGS. 1 to 4. Here, as the positive control peptide, a peptide consisting of the sequence of 58^{th} to 66^{th} amino acids in the M1 protein, which is one of the proteins expressed by influenza virus, was employed.

As illustrated in FIGS. 1 and 2, for N5 (N5-1 and N5-2; the same experiment was repeated twice using the same person's blood), it was identified that the dendritic cells transformed with the epitope group 3, 7, 9, 11, 12, 15, 18, or 21 resulted in increased expression of IFN-γ in T cells.

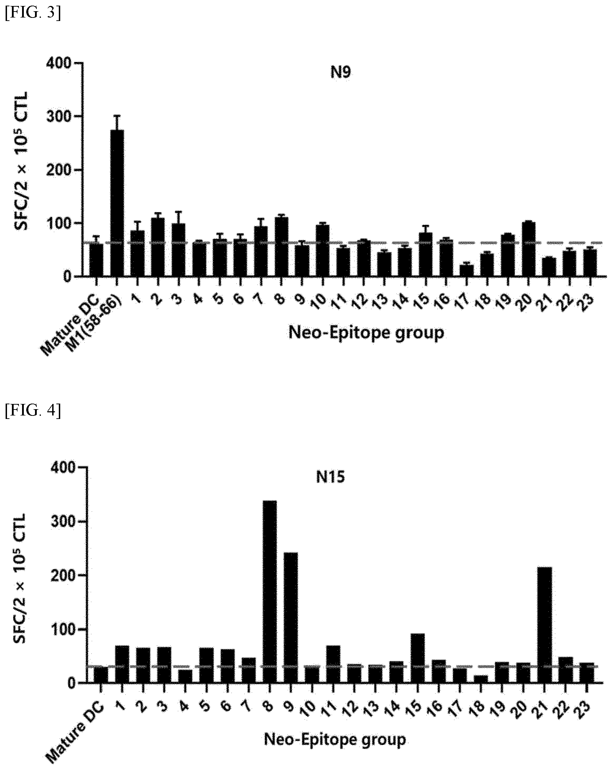

As illustrated in FIG. 3, for N9, it was identified that the dendritic cells transformed with the epitope group 1, 2, 3, 7, 8, 10, 15, 16, 19, or 20 resulted in increased expression of IFN-γ in T cells.

In addition, as illustrated in FIG. 4, for N15, it was identified that the dendritic cells transformed with the epitope group 1, 2, 3, 5, 6, 7, 8, 9, 11, 15, 21, or 22 resulted in increased expression of IFN-γ in T cells.

From these results, it was found that in the present invention, cytotoxic T lymphocytes (CTLs) can be activated by dendritic cells loaded with epitopes selected from the epitopes as set forth in Tables 1 and 2 above, and the thus activated T cells have antigen specificity which enables recognition of the epitope that is a novel antigen.

### [Example 3] ELISPOT results for T cells activated with dendritic cells loaded with selected EBNA-1 epitope

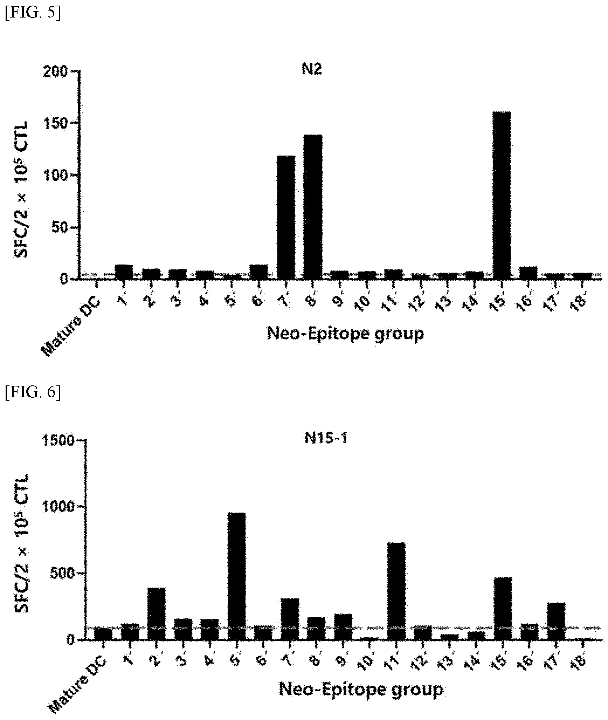

Using PBMCs extracted from the blood of three healthy humans (N2, N15, or N19), the degree of T cell activation was checked in the same manner as in Example 2, and the results are illustrated in FIGS. 5 to 8. Here, the monocytes having differentiated into dendritic cells were transformed with each group containing 10 or 11 epitope peptides as set forth in Table 4.

**[Table 4]**

| Group | SEQ ID NO |
|---|---|
| 1' | SEQ ID NO: 123, SEQ ID NO: 132, SEQ ID NO: 141, SEQ ID NO: 150, SEQ ID NO: 159, SEQ ID NO: 168, SEQ ID NO: 177, SEQ ID NO: 186, SEQ ID NO: 195 |
| 2' | SEQ ID NO: 124, SEQ ID NO: 133, SEQ ID NO: 142, SEQ ID NO: 151, SEQ ID NO: 160, SEQ ID NO: 169, SEQ ID NO: 178, SEQ ID NO: 187, SEQ ID NO: 196 |
| 3' | SEQ ID NO: 125, SEQ ID NO: 134, SEQ ID NO: 143, SEQ ID NO: 152, SEQ ID NO: 161, SEQ ID NO: 170, SEQ ID NO: 179, SEQ ID NO: 188, SEQ ID NO: 197 |
| 4' | SEQ ID NO: 126, SEQ ID NO: 135, SEQ ID NO: 144, SEQ ID NO: 153, SEQ ID NO: 162, SEQ ID NO: 171, SEQ ID NO: 180, SEQ ID NO: 189, SEQ ID NO: 198 |
| 5' | SEQ ID NO: 127, SEQ ID NO: 136, SEQ ID NO: 145, SEQ ID NO: 154, SEQ ID NO: 163, SEQ ID NO: 172, SEQ ID NO: 181, SEQ ID NO: 190, SEQ ID NO: 199 |
| 6' | SEQ ID NO: 128, SEQ ID NO: 137, SEQ ID NO: 146, SEQ ID NO: 155, SEQ ID NO: 164, SEQ ID NO: 173, SEQ ID NO: 182, SEQ ID NO: 191, SEQ ID NO: 200 |
| 7' | SEQ ID NO: 129, SEQ ID NO: 138, SEQ ID NO: 147, SEQ ID NO: 156, SEQ ID NO: 165, SEQ ID NO: 174, SEQ ID NO: 183, SEQ ID NO: 192 |
| 8' | SEQ ID NO: 130, SEQ ID NO: 139, SEQ ID NO: 148, SEQ ID NO: 157, SEQ ID NO: 166, SEQ ID NO: 175, SEQ ID NO: 184, SEQ ID NO: 193 |
| 9' | SEQ ID NO: 131, SEQ ID NO: 140, SEQ ID NO: 149, SEQ ID NO: 158, SEQ ID NO: 167, SEQ ID NO: 176, SEQ ID NO: 185, SEQ ID NO: 194 |
| 10' | SEQ ID NO: 123 to SEQ ID NO: 131 |
| 11' | SEQ ID NO: 132 to SEQ ID NO: 140 |
| 12' | SEQ ID NO: 141 to SEQ ID NO: 149 |
| 13' | SEQ ID NO: 150 to SEQ ID NO: 158 |
| 14' | SEQ ID NO: 159 to SEQ ID NO: 167 |
| 15' | SEQ ID NO: 168 to SEQ ID NO: 176 |
| 16' | SEQ ID NO: 177 to SEQ ID NO: 185 |
| 17' | SEQ ID NO: 186 to SEQ ID NO: 194 |
| 18' | SEQ ID NO: 195 to SEQ ID NO: 200 |

As illustrated in FIG. 5, for N2, it was identified that cytotoxic T lymphocytes were activated by the dendritic cells transformed with the epitope group 6', 7', 8', or 15'.

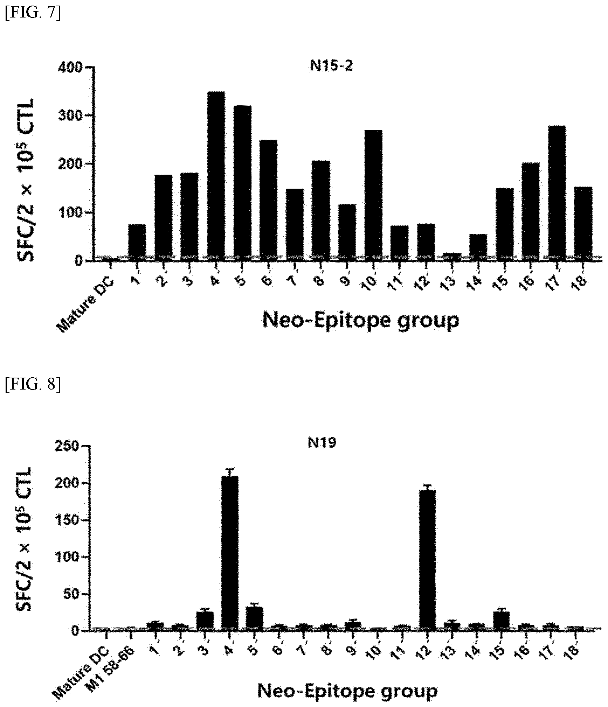

In addition, as illustrated in FIGS. 6 and 7, for N15 (N15-1 and N15-2; the same experiment was repeated twice using the same person's blood), it was identified that cytotoxic T lymphocytes were activated by the dendritic cells transformed with each of all epitope groups except for some groups.

In addition, as illustrated in FIG. 8, for N19, it was identified that cytotoxic T lymphocytes were activated by the dendritic cells transformed with the epitope group 3', 4', 5', 9', 12', or 15'.

From the results of Examples 2 and 3, it can be seen that the epitopes according to the present invention can very effectively activate cytotoxic T lymphocytes.

Although specific parts of the present invention have been described in detail, it is obvious to those skilled in the art that such a specific description is merely a preferred embodiment, and the scope of the present invention is not limited thereto. Therefore, the substantial scope of the present invention will be defined by the appended claims and equivalents thereof.

### Industrial Applicability

The present invention relates to a cancer-specific tumor antigen epitope, an antigen-presenting cell loaded with the epitope, and a method for activating T cells for cancer treatment by the antigen-presenting cell.

## Claims

1. A cancer-specific tumor antigen epitope, represented by any one of SEQ ID NOs: 1 to 3, SEQ ID NOs: 7 to 9, SEQ ID NOs: 11 to 15, SEQ ID NOs: 19 to 21, SEQ ID NO: 23 to 39, SEQ ID NO: 43 to 45, SEQ ID NO: 47 to 51, SEQ ID NO: 55 to 57, SEQ ID NO: 59 to 63, SEQ ID NO: 67 to 69, SEQ ID NO: 71 to 75, SEQ ID NO: 79 to 81, SEQ ID NO: 83 to 111, SEQ ID NOs: 115 to 117, SEQ ID NOs: 119 to 122, SEQ ID NOs: 124 to 127, SEQ ID NOs: 129 to 149, SEQ ID NOs: 151 to 154, SEQ ID NOs: 156 to 158, SEQ ID NOs: 160 to 163, SEQ ID NOs: 165 to 167, SEQ ID NOs: 168 to 176, SEQ ID NOs: 178 to 181, SEQ ID NOs: 183 to 194, and SEQ ID NOs: 196 to 199.

2. The cancer-specific tumor antigen epitope according to claim 1,
wherein the cancer-specific tumor antigen epitope exhibits binding affinity with at least one of HLA-A, HLA-B, HLA-C, HLA-E, HLA-F, HLA-G, β2-microglobulin, HLA-DPA1, HLA-DPB1, HLA-DQA1, HLA-DQB1, HLA-DRA1, HLA-DRB1, HLA-DRB3, HLA-DRB4, HLA-DRB5, HLA-DM, HLA-DOA, and HLA-DOB loci.

3. The cancer-specific tumor antigen epitope according to claim 1,
wherein the cancer-specific tumor antigen epitope exhibits binding affinity with at least one of HLA-A^{∗}2402, HLA-A^{∗}A0201, HLA-A^{∗}3303, HLA-A^{∗}1101, HLA-A^{∗}0206, HLA-A^{∗}3101, HLA-B^{∗}5101, HLA-B^{∗}4403, HLA-B^{∗}5401, HLA-B^{∗}5801, and HLA-B^{∗}3501.

4. The cancer-specific tumor antigen epitope according to claim 1,
wherein the cancer is Epstein-Barr virus (EBV)-positive cancer.

5. The cancer-specific tumor antigen epitope according to claim 4,
wherein the cancer is EBV-positive gastric cancer, EBV-positive cervical cancer, EBV-positive Burkitt's lymphoma, EBV-positive T cell lymphoma, EBV-positive breast cancer, EBV-positive leiomyosarcoma, EBV-positive smooth muscle tumor, EBV-positive Hodgkin lymphoma, EBV-positive nasopharyngeal cancer, or EBV-positive post-transplant lymphoproliferative disorder (PTLD).

6. A nucleic acid molecule, encoding the cancer-specific tumor antigen epitope according to any one of claims 1 to 5.

7. An expression vector, into which the nucleic acid molecule according to claim 6 is inserted.

8. A host cell, transfected with the expression vector according to claim 7.

9. A composition for activating T cells, comprising:
a cancer-specific tumor antigen epitope, represented by any one of SEQ ID NOs: 1 to 3, SEQ ID NOs: 7 to 9, SEQ ID NOs: 11 to 15, SEQ ID NOs: 19 to 21, SEQ ID NO: 23 to 39, SEQ ID NO: 43 to 45, SEQ ID NO: 47 to 51, SEQ ID NO: 55 to 57, SEQ ID NO: 59 to 63, SEQ ID NO: 67 to 69, SEQ ID NO: 71 to 75, SEQ ID NO: 79 to 81, SEQ ID NO: 83 to 111, SEQ ID NOs: 115 to 117, SEQ ID NOs: 119 to 122, SEQ ID NOs: 124 to 127, SEQ ID NOs: 129 to 149, SEQ ID NOs: 151 to 154, SEQ ID NOs: 156 to 158, SEQ ID NOs: 160 to 163, SEQ ID NOs: 165 to 167, SEQ ID NOs: 168 to 176, SEQ ID NOs: 178 to 181, SEQ ID NOs: 183 to 194, and SEQ ID NOs: 196 to 199.

10. The composition according to claim 9,
wherein the T cells are for prevention or treatment of cancer.

11. The composition according to claim 9,
wherein the T cells include one or more selected from the group consisting of cytotoxic T cells, helper T cells, natural killer T cells, γδ T cells, regulatory T cells, and memory T cells.

12. An antigen-presenting cell, loaded with a cancer-specific tumor antigen epitope represented by any one of the following:
SEQ ID NOs: 1 to 3, SEQ ID NOs: 7 to 9, SEQ ID NOs: 11 to 15, SEQ ID NOs: 19 to 21, SEQ ID NO: 23 to 39, SEQ ID NO: 43 to 45, SEQ ID NO: 47 to 51, SEQ ID NO: 55 to 57, SEQ ID NO: 59 to 63, SEQ ID NO: 67 to 69, SEQ ID NO: 71 to 75, SEQ ID NO: 79 to 81, SEQ ID NO: 83 to 111, SEQ ID NOs: 115 to 117, SEQ ID NOs: 119 to 122, SEQ ID NOs: 124 to 127, SEQ ID NOs: 129 to 149, SEQ ID NOs: 151 to 154, SEQ ID NOs: 156 to 158, SEQ ID NOs: 160 to 163, SEQ ID NOs: 165 to 167, SEQ ID NOs: 168 to 176, SEQ ID NOs: 178 to 181, SEQ ID NOs: 183 to 194, and SEQ ID NOs: 196 to 199.

13. The antigen-presenting cell according to claim 12,
wherein the antigen-presenting cell includes one or more of dendritic cell, B cell, and macrophage.

14. The antigen-presenting cell according to claim 13,
wherein the antigen-presenting cell promotes proliferation or differentiation of T cells.

15. A fusion protein, comprising:
a cancer-specific tumor antigen epitope, represented by any one of SEQ ID NOs: 1 to 3, SEQ ID NOs: 7 to 9, SEQ ID NOs: 11 to 15, SEQ ID NOs: 19 to 21, SEQ ID NO: 23 to 39, SEQ ID NO: 43 to 45, SEQ ID NO: 47 to 51, SEQ ID NO: 55 to 57, SEQ ID NO: 59 to 63, SEQ ID NO: 67 to 69, SEQ ID NO: 71 to 75, SEQ ID NO: 79 to 81, SEQ ID NO: 83 to 111, SEQ ID NOs: 115 to 117, SEQ ID NOs: 119 to 122, SEQ ID NOs: 124 to 127, SEQ ID NOs: 129 to 149, SEQ ID NOs: 151 to 154, SEQ ID NOs: 156 to 158, SEQ ID NOs: 160 to 163, SEQ ID NOs: 165 to 167, SEQ ID NOs: 168 to 176, SEQ ID NOs: 178 to 181, SEQ ID NOs: 183 to 194, and SEQ ID NOs: 196 to 199; and
a dendritic cell-specific antibody or a fragment thereof.

16. The fusion protein according to claim 15,
wherein the dendritic cell-specific antibody is an antibody specific for DCIR, MHC class I, MHC class II, CD1, CD2, CD3, CD4, CD8, CD11b, CD14, CD15, CD16, CD19, CD20, CD29, CD31, CD40, CD43, CD44, CD45, CD54, CD56, CD57, CD58, CD83, CD86, CMRF-44, CMRF-56, DCIR, DC-ASPGR, CLEC-6, CD40, BDCA-2, MARCO, DEC-205, Clec9A, 33D1, mannose receptor, Langerin, DECTIN-1, B7-1, B7-2, IFN-γ receptor, IL-2 receptor, ICAM-1, Fcγ receptor, LOX-1, or ASPGR, which is on dendritic cells.

17. A method for producing an antigen-presenting cell, wherein the antigen-presenting cell is loaded with a cancer-specific tumor antigen epitope represented by any one of the following:
SEQ ID NOs: 1 to 3, SEQ ID NOs: 7 to 9, SEQ ID NOs: 11 to 15, SEQ ID NOs: 19 to 21, SEQ ID NO: 23 to 39, SEQ ID NO: 43 to 45, SEQ ID NO: 47 to 51, SEQ ID NO: 55 to 57, SEQ ID NO: 59 to 63, SEQ ID NO: 67 to 69, SEQ ID NO: 71 to 75, SEQ ID NO: 79 to 81, SEQ ID NO: 83 to 111, SEQ ID NOs: 115 to 117, SEQ ID NOs: 119 to 122, SEQ ID NOs: 124 to 127, SEQ ID NOs: 129 to 149, SEQ ID NOs: 151 to 154, SEQ ID NOs: 156 to 158, SEQ ID NOs: 160 to 163, SEQ ID NOs: 165 to 167, SEQ ID NOs: 168 to 176, SEQ ID NOs: 178 to 181, SEQ ID NOs: 183 to 194, and SEQ ID NOs: 196 to 199.

18. The method according to claim 17,
wherein the antigen-presenting cell includes one or more of dendritic cell, B cell, and macrophage.

19. The method according to claim 17,
wherein the antigen-presenting cell is obtained from peripheral blood mononuclear cells (PBMCs) derived from peripheral blood of a target individual.

20. The method according to claim 17,
wherein the loading is performed by contacting the antigen-presenting cell with the cancer-specific tumor antigen epitope.

21. The method according to claim 17,
wherein the loading is performed by pulsing the antigen-presenting cell with the cancer-specific tumor antigen epitope.

22. The method according to claim 17,
wherein the loading is performed by nucleofection of the antigen-presenting cell with an expression vector into which a nucleic acid molecule encoding the cancer-specific tumor antigen epitope is inserted.

23. The method according to claim 17,
wherein the loading is performed using a fusion protein that contains the cancer-specific tumor antigen epitope; and a dendritic cell-specific antibody or a fragment thereof.

24. A T cell activated by the antigen-presenting cell according to any one of claims 12 to 14.

25. A method for activating T cells using the antigen-presenting cell according to any one of claims 12 to 14.

26. The method according to claim 25,
wherein the method is performed by co-culture of the T cells with the antigen-presenting cell.

27. The method according to claim 25,
wherein the T cells are obtained from peripheral blood mononuclear cells (PBMCs) of a target individual.

28. The method according to claim 25,
wherein the T cells include one or more selected from the group consisting of cytotoxic T cells, helper T cells, natural killer T cells, γδ T cells, regulatory T cells, and memory T cells.

29. The method according to claim 26,
wherein the co-culture is performed with addition of interleukin-2 (IL-2), interleukin-4 (IL-4), interleukin-7 (IL-7), interleukin-15 (IL-15), interleukin-21 (IL-21), or a combination thereof.

30. The method according to claim 26,
wherein the co-culture is performed with addition of a fusion protein that contains a cytokine and an immunoglobulin heavy chain constant region.

31. The method according to claim 30,
wherein the cytokine is interferon-γ (IFN-γ), interleukin-2 (IL-2), interleukin-4 (IL-4), interleukin-12 (IL-12), IL-18, tumor necrosis factor (TNF), or granulocyte macrophage colony stimulating factor (GMCSF).

32. The method according to claim 26,
wherein the co-culture is performed with addition of a fusion protein that contains ligand of CD27, CXCR3, or CD62L; and an immunoglobulin heavy chain constant region.

33. A pharmaceutical composition for preventing or treating cancer, comprising as an active ingredient:
the antigen-presenting cell according to any one of claims 12 to 14.

34. The pharmaceutical composition according to claim 33,
wherein the cancer is Epstein-Barr virus (EBV)-positive cancer.

35. A pharmaceutical composition for preventing or treating cancer, comprising as an active ingredient:
the activated T cell according to claim 24.

36. The pharmaceutical composition according to claim 35,
wherein the cancer is Epstein-Barr virus (EBV)-positive cancer.


Sequence List Free Text

<110>	Good T Cells, Inc.
<120>	A Method for activating T cells to treat cancer
<130>	POPB201876PCT
<150>	KR 10-2019-0015108
<151>	2019-02-08
<160>	200
<170>	KoPatentIn 3.0

<210>	1
<211>	15
<212>	PRT
<213>	Artificial Sequence
<220>
<223>	LMP2a epitope
<400>	1
Met Gly Ser Leu Glu Met Val Pro Met Gly Ala Gly Pro Pro Ser
 1               5                   10                  15

<210>	2

<211>    15
<212>    PRT
<213>    Artificial Sequence
<220>
<223>    LMP2a epitope
<400>    2
Glu Met Val Pro Met Gly Ala Gly Pro Pro Ser Pro Gly Gly Asp
1               5                   10                  15

<210>    3
<211>    15
<212>    PRT
<213>    Artificial Sequence
<220>
<223>    LMP2a epitope
<400>    3
Met Gly Ala Gly Pro Pro Ser Pro Gly Gly Asp Pro Asp Gly Asp
1               5                   10                  15

<210>    4
<211>    15
<212>    PRT
<213>    Artificial Sequence
<220>
<223>    LMP2a epitope
<400>    4
Pro Pro Ser Pro Gly Gly Asp Pro Asp Gly Asp Asp Gly Gly Asn
1               5                   10                  15

<210>    5
<211>    15
<212>    PRT
<213>    Artificial Sequence
<220>
<223>    LMP2a epitope
<400>    5
Gly Gly Asp Pro Asp Gly Asp Asp Gly Gly Asn Asn Ser Gln Tyr
1               5                   10                  15

<210>    6
<211>    15
<212>    PRT
<213>    Artificial Sequence
<220>
<223>    LMP2a epitope
<400>    6
Asp Gly Asp Asp Gly Gly Asn Asn Ser Gln Tyr Pro Ser Ala Ser
1               5                   10                  15

<210>    7
<211>    15

<212>    PRT
<213>    Artificial Sequence
<220>
<223>    LMP2a epitope
<400>    7
Gly Gly Asn Asn Ser Gln Tyr Pro Ser Ala Ser Gly Ser Tyr Gly
1               5                   10                  15

<210>    8
<211>    15
<212>    PRT
<213>    Artificial Sequence
<220>
<223>    LMP2a epitope
<400>    8
Ser Gln Tyr Pro Ser Ala Ser Gly Ser Tyr Gly Asn Thr Pro Thr
1               5                   10                  15

<210>    9
<211>    15
<212>    PRT
<213>    Artificial Sequence
<220>
<223>    LMP2a epitope
<400>    9
Ser Ala Ser Gly Ser Tyr Gly Asn Thr Pro Thr Pro Pro Asn Asp
1               5                   10                  15

<210>    10
<211>    15
<212>    PRT
<213>    Artificial Sequence
<220>
<223>    LMP2a epitope
<400>    10
Ser Tyr Gly Asn Thr Pro Thr Pro Pro Asn Asp Glu Glu Arg Glu
1               5                   10                  15

<210>    11
<211>    15
<212>    PRT
<213>    Artificial Sequence
<220>
<223>    LMP2a epitope
<400>    11
Thr Pro Thr Pro Pro Asn Asp Glu Glu Arg Glu Ser Asn Glu Glu
1               5                   10                  15

<210>    12
<211>    15
<212>    PRT

<213>    Artificial Sequence
<220>
<223>    LMP2a epitope
<400>    12
Pro Asn Asp Glu Glu Arg Glu Ser Asn Glu Glu Pro Pro Pro Pro
1               5                   10                  15

<210>    13
<211>    15
<212>    PRT
<213>    Artificial Sequence
<220>
<223>    LMP2a epitope
<400>    13
Glu Arg Glu Ser Asn Glu Glu Pro Pro Pro Pro Tyr Glu Asp Leu
1               5                   10                  15

<210>    14
<211>    15
<212>    PRT
<213>    Artificial Sequence
<220>
<223>    LMP2a epitope
<400>    14
Asn Glu Glu Pro Pro Pro Pro Tyr Glu Asp Leu Asp Trp Gly Asn
1               5                   10                  15

<210>    15
<211>    15
<212>    PRT
<213>    Artificial Sequence
<220>
<223>    LMP2a epitope
<400>    15
Pro Pro Pro Tyr Glu Asp Leu Asp Trp Gly Asn Gly Asp Arg His
1               5                   10                  15

<210>    16
<211>    15
<212>    PRT
<213>    Artificial Sequence
<220>
<223>    LMP2a epitope
<400>    16
Glu Asp Leu Asp Trp Gly Asn Gly Asp Arg His Ser Asp Tyr Gln
1               5                   10                  15

<210>    17
<211>    15
<212>    PRT
<213>    Artificial Sequence

<220>
<223>    LMP2a epitope
<400>    17
Trp Gly Asn Gly Asp Arg His Ser Asp Tyr Gln Pro Leu Gly Asn
1               5                   10                  15

<210>    18
<211>    15
<212>    PRT
<213>    Artificial Sequence
<220>
<223>    LMP2a epitope
<400>    18
Asp Arg His Ser Asp Tyr Gln Pro Leu Gly Asn Gln Asp Pro Ser
1               5                   10                  15

<210>    19
<211>    15
<212>    PRT
<213>    Artificial Sequence
<220>
<223>    LMP2a epitope
<400>    19
Asp Tyr Gln Pro Leu Gly Asn Gln Asp Pro Ser Leu Tyr Leu Gly
1               5                   10                  15

<210>    20
<211>    15
<212>    PRT
<213>    Artificial Sequence
<220>
<223>    LMP2a epitope
<400>    20
Leu Gly Asn Gln Asp Pro Ser Leu Tyr Leu Gly Leu Gln His Asp
1               5                   10                  15

<210>    21
<211>    15
<212>    PRT
<213>    Artificial Sequence
<220>
<223>    LMP2a epitope
<400>    21
Asp Pro Ser Leu Tyr Leu Gly Leu Gln His Asp Gly Asn Asp Gly
1               5                   10                  15

<210>    22
<211>    15
<212>    PRT
<213>    Artificial Sequence
<220>

<223>    LMP2a epitope
<400>    22
Tyr Leu Gly Leu Gln His Asp Gly Asn Asp Gly Leu Pro Pro Pro
1               5                   10                  15

<210>    23
<211>    15
<212>    PRT
<213>    Artificial Sequence
<220>
<223>    LMP2a epitope
<400>    23
Gln His Asp Gly Asn Asp Gly Leu Pro Pro Pro Pro Tyr Ser Pro
1               5                   10                  15

<210>    24
<211>    15
<212>    PRT
<213>    Artificial Sequence
<220>
<223>    LMP2a epitope
<400>    24
Asn Asp Gly Leu Pro Pro Pro Pro Tyr Ser Pro Arg Asp Asp Ser
1               5                   10                  15

<210>    25
<211>    15
<212>    PRT
<213>    Artificial Sequence
<220>
<223>    LMP2a epitope
<400>    25
Pro Pro Pro Pro Tyr Ser Pro Arg Asp Asp Ser Ser Gln His Ile
1               5                   10                  15

<210>    26
<211>    15
<212>    PRT
<213>    Artificial Sequence
<220>
<223>    LMP2a epitope
<400>    26
Tyr Ser Pro Arg Asp Asp Ser Ser Gln His Ile Tyr Glu Glu Ala
1               5                   10                  15

<210>    27
<211>    15
<212>    PRT
<213>    Artificial Sequence
<220>
<223>    LMP2a epitope

<400>    27
Asp Asp Ser Ser Gln His Ile Tyr Glu Glu Ala Gly Arg Gly Ser
1               5                   10                  15

<210>    28
<211>    15
<212>    PRT
<213>    Artificial Sequence
<220>
<223>    LMP2a epitope
<400>    28
Gln His Ile Tyr Glu Glu Ala Gly Arg Gly Ser Met Asn Pro Val
1               5                   10                  15

<210>    29
<211>    15
<212>    PRT
<213>    Artificial Sequence
<220>
<223>    LMP2a epitope
<400>    29
Glu Glu Ala Gly Arg Gly Ser Met Asn Pro Val Cys Leu Pro Val
1               5                   10                  15

<210>    30
<211>    15
<212>    PRT
<213>    Artificial Sequence
<220>
<223>    LMP2a epitope
<400>    30
Arg Gly Ser Met Asn Pro Val Cys Leu Pro Val Ile Val Ala Pro
1               5                   10                  15

<210>    31
<211>    15
<212>    PRT
<213>    Artificial Sequence
<220>
<223>    LMP2a epitope
<400>    31
Asn Pro Val Cys Leu Pro Val Ile Val Ala Pro Tyr Leu Phe Trp
1               5                   10                  15

<210>    32
<211>    15
<212>    PRT
<213>    Artificial Sequence
<220>
<223>    LMP2a epitope
<400>    32

Leu Pro Val Ile Val Ala Pro Tyr Leu Phe Trp Leu Ala Ala Ile
1               5                   10                  15

<210>   33
<211>   15
<212>   PRT
<213>   Artificial Sequence
<220>
<223>   LMP2a epitope
<400>   33
Val Ala Pro Tyr Leu Phe Trp Leu Ala Ala Ile Ala Ala Ser Cys
1               5                   10                  15

<210>   34
<211>   15
<212>   PRT
<213>   Artificial Sequence
<220>
<223>   LMP2a epitope
<400>   34
Leu Phe Trp Leu Ala Ala Ile Ala Ala Ser Cys Phe Thr Ala Ser
1               5                   10                  15

<210>   35
<211>   15
<212>   PRT
<213>   Artificial Sequence
<220>
<223>   LMP2a epitope
<400>   35
Ala Ala Ile Ala Ala Ser Cys Phe Thr Ala Ser Val Ser Thr Val
1               5                   10                  15

<210>   36
<211>   15
<212>   PRT
<213>   Artificial Sequence
<220>
<223>   LMP2a epitope
<400>   36
Ala Ser Cys Phe Thr Ala Ser Val Ser Thr Val Val Thr Ala Thr
1               5                   10                  15

<210>   37
<211>   15
<212>   PRT
<213>   Artificial Sequence
<220>
<223>   LMP2a epitope
<400>   37
Thr Ala Ser Val Ser Thr Val Val Thr Ala Thr Gly Leu Ala Leu

1               5                   10                  15

<210>   38
<211>   15
<212>   PRT
<213>   Artificial Sequence
<220>
<223>   LMP2a epitope
<400>   38
Ser Thr Val Val Thr Ala Thr Gly Leu Ala Leu Ser Leu Leu Leu
1               5                   10                  15

<210>   39
<211>   15
<212>   PRT
<213>   Artificial Sequence
<220>
<223>   LMP2a epitope
<400>   39
Thr Ala Thr Gly Leu Ala Leu Ser Leu Leu Leu Leu Ala Ala Val
1               5                   10                  15

<210>   40
<211>   15
<212>   PRT
<213>   Artificial Sequence
<220>
<223>   LMP2a epitope
<400>   40
Leu Ala Leu Ser Leu Leu Leu Leu Ala Ala Val Ala Ser Ser Tyr
1               5                   10                  15

<210>   41
<211>   15
<212>   PRT
<213>   Artificial Sequence
<220>
<223>   LMP2a epitope
<400>   41
Leu Leu Leu Leu Ala Ala Val Ala Ser Ser Tyr Ala Ala Ala Gln
1               5                   10                  15

<210>   42
<211>   15
<212>   PRT
<213>   Artificial Sequence
<220>
<223>   LMP2a epitope
<400>   42
Ala Ala Val Ala Ser Ser Tyr Ala Ala Ala Gln Arg Lys Leu Leu
1               5                   10                  15

<210> 43
<211> 15
<212> PRT
<213> Artificial Sequence
<220>
<223> LMP2a epitope
<400> 43
Ser Ser Tyr Ala Ala Ala Gln Arg Lys Leu Leu Thr Pro Val Thr
1               5                   10                  15

<210> 44
<211> 15
<212> PRT
<213> Artificial Sequence
<220>
<223> LMP2a epitope
<400> 44
Ala Ala Gln Arg Lys Leu Leu Thr Pro Val Thr Val Leu Thr Ala
1               5                   10                  15

<210> 45
<211> 15
<212> PRT
<213> Artificial Sequence
<220>
<223> LMP2a epitope
<400> 45
Lys Leu Leu Thr Pro Val Thr Val Leu Thr Ala Val Val Thr Phe
1               5                   10                  15

<210> 46
<211> 15
<212> PRT
<213> Artificial Sequence
<220>
<223> LMP2a epitope
<400> 46
Pro Val Thr Val Leu Thr Ala Val Val Thr Phe Phe Ala Ile Cys
1               5                   10                  15

<210> 47
<211> 15
<212> PRT
<213> Artificial Sequence
<220>
<223> LMP2a epitope
<400> 47
Leu Thr Ala Val Val Thr Phe Phe Ala Ile Cys Leu Thr Trp Arg
1               5                   10                  15

<210>  48
<211>  15
<212>  PRT
<213>  Artificial Sequence
<220>
<223>  LMP2a epitope
<400>  48
Val Thr Phe Phe Ala Ile Cys Leu Thr Trp Arg Ile Glu Asp Pro
1               5                   10                  15

<210>  49
<211>  15
<212>  PRT
<213>  Artificial Sequence
<220>
<223>  LMP2a epitope
<400>  49
Ala Ile Cys Leu Thr Trp Arg Ile Glu Asp Pro Pro Phe Asn Ser
1               5                   10                  15

<210>  50
<211>  15
<212>  PRT
<213>  Artificial Sequence
<220>
<223>  LMP2a epitope
<400>  50
Thr Trp Arg Ile Glu Asp Pro Pro Phe Asn Ser Leu Leu Phe Ala
1               5                   10                  15

<210>  51
<211>  15
<212>  PRT
<213>  Artificial Sequence
<220>
<223>  LMP2a epitope
<400>  51
Glu Asp Pro Pro Phe Asn Ser Leu Leu Phe Ala Leu Leu Ala Ala
1               5                   10                  15

<210>  52
<211>  15
<212>  PRT
<213>  Artificial Sequence
<220>
<223>  LMP2a epitope
<400>  52
Phe Asn Ser Leu Leu Phe Ala Leu Leu Ala Ala Ala Gly Gly Leu
1               5                   10                  15

<210>  53

<211>    15
<212>    PRT
<213>    Artificial Sequence
<220>
<223>    LMP2a epitope
<400>    53
Leu Phe Ala Leu Leu Ala Ala Ala Gly Gly Leu Gln Gly Ile Tyr
1               5                   10                  15

<210>    54
<211>    15
<212>    PRT
<213>    Artificial Sequence
<220>
<223>    LMP2a epitope
<400>    54
Leu Ala Ala Ala Gly Gly Leu Gln Gly Ile Tyr Val Leu Val Met
1               5                   10                  15

<210>    55
<211>    15
<212>    PRT
<213>    Artificial Sequence
<220>
<223>    LMP2a epitope
<400>    55
Gly Gly Leu Gln Gly Ile Tyr Val Leu Val Met Leu Val Leu Leu
1               5                   10                  15

<210>    56
<211>    15
<212>    PRT
<213>    Artificial Sequence
<220>
<223>    LMP2a epitope
<400>    56
Gly Ile Tyr Val Leu Val Met Leu Val Leu Leu Ile Leu Ala Tyr
1               5                   10                  15

<210>    57
<211>    15
<212>    PRT
<213>    Artificial Sequence
<220>
<223>    LMP2a epitope
<400>    57
Leu Val Met Leu Val Leu Leu Ile Leu Ala Tyr Arg Arg Arg Trp
1               5                   10                  15

<210>    58
<211>    15

<212>    PRT
<213>    Artificial Sequence
<220>
<223>    LMP2a epitope
<400>    58
Val Leu Leu Ile Leu Ala Tyr Arg Arg Arg Trp Arg Arg Leu Thr
1               5                   10                  15

<210>    59
<211>    15
<212>    PRT
<213>    Artificial Sequence
<220>
<223>    LMP2a epitope
<400>    59
Leu Ala Tyr Arg Arg Arg Trp Arg Arg Leu Thr Val Cys Gly Gly
1               5                   10                  15

<210>    60
<211>    15
<212>    PRT
<213>    Artificial Sequence
<220>
<223>    LMP2a epitope
<400>    60
Arg Arg Trp Arg Arg Leu Thr Val Cys Gly Gly Ile Met Phe Leu
1               5                   10                  15

<210>    61
<211>    15
<212>    PRT
<213>    Artificial Sequence
<220>
<223>    LMP2a epitope
<400>    61
Arg Leu Thr Val Cys Gly Gly Ile Met Phe Leu Ala Cys Val Leu
1               5                   10                  15

<210>    62
<211>    15
<212>    PRT
<213>    Artificial Sequence
<220>
<223>    LMP2a epitope
<400>    62
Cys Gly Gly Ile Met Phe Leu Ala Cys Val Leu Val Leu Ile Val
1               5                   10                  15

<210>    63
<211>    15
<212>    PRT

<213>    Artificial Sequence
<220>
<223>    LMP2a epitope
<400>    63
Met Phe Leu Ala Cys Val Leu Val Leu Ile Val Asp Ala Val Leu
1               5                   10                  15

<210>    64
<211>    15
<212>    PRT
<213>    Artificial Sequence
<220>
<223>    LMP2a epitope
<400>    64
Cys Val Leu Val Leu Ile Val Asp Ala Val Leu Gln Leu Ser Pro
1               5                   10                  15

<210>    65
<211>    15
<212>    PRT
<213>    Artificial Sequence
<220>
<223>    LMP2a epitope
<400>    65
Leu Ile Val Asp Ala Val Leu Gln Leu Ser Pro Leu Leu Gly Ala
1               5                   10                  15

<210>    66
<211>    15
<212>    PRT
<213>    Artificial Sequence
<220>
<223>    LMP2a epitope
<400>    66
Ala Val Leu Gln Leu Ser Pro Leu Leu Gly Ala Val Thr Val Val
1               5                   10                  15

<210>    67
<211>    15
<212>    PRT
<213>    Artificial Sequence
<220>
<223>    LMP2a epitope
<400>    67
Leu Ser Pro Leu Leu Gly Ala Val Thr Val Val Ser Met Thr Leu
1               5                   10                  15

<210>    68
<211>    15
<212>    PRT
<213>    Artificial Sequence

<220>
<223>    LMP2a epitope
<400>    68
Leu Gly Ala Val Thr Val Val Ser Met Thr Leu Leu Leu Leu Ala
1               5                   10                  15

<210>    69
<211>    15
<212>    PRT
<213>    Artificial Sequence
<220>
<223>    LMP2a epitope
<400>    69
Thr Val Val Ser Met Thr Leu Leu Leu Leu Ala Phe Val Leu Trp
1               5                   10                  15

<210>    70
<211>    15
<212>    PRT
<213>    Artificial Sequence
<220>
<223>    LMP2a epitope
<400>    70
Met Thr Leu Leu Leu Leu Ala Phe Val Leu Trp Leu Ser Ser Pro
1               5                   10                  15

<210>    71
<211>    15
<212>    PRT
<213>    Artificial Sequence
<220>
<223>    LMP2a epitope
<400>    71
Leu Leu Ala Phe Val Leu Trp Leu Ser Ser Pro Gly Gly Leu Gly
1               5                   10                  15

<210>    72
<211>    15
<212>    PRT
<213>    Artificial Sequence
<220>
<223>    LMP2a epitope
<400>    72
Val Leu Trp Leu Ser Ser Pro Gly Gly Leu Gly Thr Leu Gly Ala
1               5                   10                  15

<210>    73
<211>    15
<212>    PRT
<213>    Artificial Sequence
<220>

<223>    LMP2a epitope
<400>    73
Ser Ser Pro Gly Gly Leu Gly Thr Leu Gly Ala Ala Leu Leu Thr
1               5                   10                  15

<210>    74
<211>    15
<212>    PRT
<213>    Artificial Sequence
<220>
<223>    LMP2a epitope
<400>    74
Gly Leu Gly Thr Leu Gly Ala Ala Leu Leu Thr Leu Ala Ala Ala
1               5                   10                  15

<210>    75
<211>    15
<212>    PRT
<213>    Artificial Sequence
<220>
<223>    LMP2a epitope
<400>    75
Leu Gly Ala Ala Leu Leu Thr Leu Ala Ala Ala Leu Ala Leu Leu
1               5                   10                  15

<210>    76
<211>    15
<212>    PRT
<213>    Artificial Sequence
<220>
<223>    LMP2a epitope
<400>    76
Leu Leu Thr Leu Ala Ala Ala Leu Ala Leu Leu Ala Ser Leu Ile
1               5                   10                  15

<210>    77
<211>    15
<212>    PRT
<213>    Artificial Sequence
<220>
<223>    LMP2a epitope
<400>    77
Ala Ala Ala Leu Ala Leu Leu Ala Ser Leu Ile Leu Gly Thr Leu
1               5                   10                  15

<210>    78
<211>    15
<212>    PRT
<213>    Artificial Sequence
<220>
<223>    LMP2a epitope

<400>    78
Ala Leu Leu Ala Ser Leu Ile Leu Gly Thr Leu Asn Leu Thr Thr
1               5                   10                  15

<210>    79
<211>    15
<212>    PRT
<213>    Artificial Sequence
<220>
<223>    LMP2a epitope
<400>    79
Ser Leu Ile Leu Gly Thr Leu Asn Leu Thr Thr Met Phe Leu Leu
1               5                   10                  15

<210>    80
<211>    15
<212>    PRT
<213>    Artificial Sequence
<220>
<223>    LMP2a epitope
<400>    80
Gly Thr Leu Asn Leu Thr Thr Met Phe Leu Leu Met Leu Leu Trp
1               5                   10                  15

<210>    81
<211>    15
<212>    PRT
<213>    Artificial Sequence
<220>
<223>    LMP2a epitope
<400>    81
Leu Thr Thr Met Phe Leu Leu Met Leu Leu Trp Thr Leu Val Val
1               5                   10                  15

<210>    82
<211>    15
<212>    PRT
<213>    Artificial Sequence
<220>
<223>    LMP2a epitope
<400>    82
Phe Leu Leu Met Leu Leu Trp Thr Leu Val Val Leu Leu Ile Cys
1               5                   10                  15

<210>    83
<211>    15
<212>    PRT
<213>    Artificial Sequence
<220>
<223>    LMP2a epitope
<400>    83

Leu Leu Trp Thr Leu Val Val Leu Leu Ile Cys Ser Ser Cys Ser
1               5                   10                  15

<210>   84
<211>   15
<212>   PRT
<213>   Artificial Sequence
<220>
<223>   LMP2a epitope
<400>   84
Leu Val Val Leu Leu Ile Cys Ser Ser Cys Ser Ser Cys Pro Leu
1               5                   10                  15

<210>   85
<211>   15
<212>   PRT
<213>   Artificial Sequence
<220>
<223>   LMP2a epitope
<400>   85
Leu Ile Cys Ser Ser Cys Ser Ser Cys Pro Leu Thr Lys Ile Leu
1               5                   10                  15

<210>   86
<211>   15
<212>   PRT
<213>   Artificial Sequence
<220>
<223>   LMP2a epitope

<400>   86
Ser Cys Ser Ser Cys Pro Leu Thr Lys Ile Leu Leu Ala Arg Leu
1               5                   10                  15
<210>   87
<211>   15
<212>   PRT
<213>   Artificial Sequence
<220>
<223>   LMP2a epitope

<400>   87
Cys Pro Leu Thr Lys Ile Leu Leu Ala Arg Leu Phe Leu Tyr Ala
1               5                   10                  15
<210>   88
<211>   15
<212>   PRT
<213>   Artificial Sequence
<220>
<223>   LMP2a epitope
<400>   88
Lys Ile Leu Leu Ala Arg Leu Phe Leu Tyr Ala Leu Ala Leu Leu

1               5                   10                  15

<210>   89
<211>   15
<212>   PRT
<213>   Artificial Sequence
<220>
<223>   LMP2a epitope
<400>   89
Ala Arg Leu Phe Leu Tyr Ala Leu Ala Leu Leu Leu Leu Ala Ser
1               5                   10                  15

<210>   90
<211>   15
<212>   PRT
<213>   Artificial Sequence
<220>
<223>   LMP2a epitope
<400>   90
Leu Tyr Ala Leu Ala Leu Leu Leu Leu Ala Ser Ala Leu Ile Ala
1               5                   10                  15

<210>   91
<211>   15
<212>   PRT
<213>   Artificial Sequence
<220>
<223>   LMP2a epitope
<400>   91
Ala Leu Leu Leu Leu Ala Ser Ala Leu Ile Ala Gly Gly Ser Ile
1               5                   10                  15

<210>   92
<211>   15
<212>   PRT
<213>   Artificial Sequence
<220>
<223>   LMP2a epitope
<400>   92
Leu Ala Ser Ala Leu Ile Ala Gly Gly Ser Ile Leu Gln Thr Asn
1               5                   10                  15

<210>   93
<211>   15
<212>   PRT
<213>   Artificial Sequence
<220>
<223>   LMP2a epitope
<400>   93
Leu Ile Ala Gly Gly Ser Ile Leu Gln Thr Asn Phe Lys Ser Leu
1               5                   10                  15

<210> 94
<211> 15
<212> PRT
<213> Artificial Sequence
<220>
<223> LMP2a epitope
<400> 94
Gly Ser Ile Leu Gln Thr Asn Phe Lys Ser Leu Ser Ser Thr Glu
1               5                   10                  15

<210> 95
<211> 15
<212> PRT
<213> Artificial Sequence
<220>
<223> LMP2a epitope
<400> 95
Gln Thr Asn Phe Lys Ser Leu Ser Ser Thr Glu Phe Ile Pro Asn
1               5                   10                  15

<210> 96
<211> 15
<212> PRT
<213> Artificial Sequence
<220>
<223> LMP2a epitope
<400> 96
Lys Ser Leu Ser Ser Thr Glu Phe Ile Pro Asn Leu Phe Cys Met
1               5                   10                  15

<210> 97
<211> 15
<212> PRT
<213> Artificial Sequence
<220>
<223> LMP2a epitope
<400> 97
Ser Thr Glu Phe Ile Pro Asn Leu Phe Cys Met Leu Leu Leu Ile
1               5                   10                  15

<210> 98
<211> 15
<212> PRT
<213> Artificial Sequence
<220>
<223> LMP2a epitope
<400> 98
Ile Pro Asn Leu Phe Cys Met Leu Leu Leu Ile Val Ala Gly Ile
1               5                   10                  15

<210> 99
<211> 15
<212> PRT
<213> Artificial Sequence
<220>
<223> LMP2a epitope
<400> 99
Phe Cys Met Leu Leu Leu Ile Val Ala Gly Ile Leu Phe Ile Leu
1               5                   10                  15

<210> 100
<211> 15
<212> PRT
<213> Artificial Sequence
<220>
<223> LMP2a epitope
<400> 100
Leu Leu Ile Val Ala Gly Ile Leu Phe Ile Leu Ala Ile Leu Thr
1               5                   10                  15

<210> 101
<211> 15
<212> PRT
<213> Artificial Sequence
<220>
<223> LMP2a epitope
<400> 101
Ala Gly Ile Leu Phe Ile Leu Ala Ile Leu Thr Glu Trp Gly Ser
1               5                   10                  15

<210> 102
<211> 15
<212> PRT
<213> Artificial Sequence
<220>
<223> LMP2a epitope
<400> 102
Phe Ile Leu Ala Ile Leu Thr Glu Trp Gly Ser Gly Asn Arg Thr
1               5                   10                  15

<210> 103
<211> 15
<212> PRT
<213> Artificial Sequence
<220>
<223> LMP2a epitope
<400> 103
Ile Leu Thr Glu Trp Gly Ser Gly Asn Arg Thr Tyr Gly Pro Val
1               5                   10                  15

<210> 104

<211>    15
<212>    PRT
<213>    Artificial Sequence
<220>
<223>    LMP2a epitope
<400>    104
Trp Gly Ser Gly Asn Arg Thr Tyr Gly Pro Val Phe Met Cys Leu
1               5                   10                  15

<210>    105
<211>    15
<212>    PRT
<213>    Artificial Sequence
<220>
<223>    LMP2a epitope
<400>    105
Asn Arg Thr Tyr Gly Pro Val Phe Met Cys Leu Gly Gly Leu Leu
1               5                   10                  15

<210>    106
<211>    15
<212>    PRT
<213>    Artificial Sequence
<220>
<223>    LMP2a epitope
<400>    106
Gly Pro Val Phe Met Cys Leu Gly Gly Leu Leu Thr Met Val Ala
1               5                   10                  15

<210>    107
<211>    15
<212>    PRT
<213>    Artificial Sequence
<220>
<223>    LMP2a epitope
<400>    107
Met Cys Leu Gly Gly Leu Leu Thr Met Val Ala Gly Ala Val Trp
1               5                   10                  15

<210>    108
<211>    15
<212>    PRT
<213>    Artificial Sequence
<220>
<223>    LMP2a epitope
<400>    108
Gly Leu Leu Thr Met Val Ala Gly Ala Val Trp Leu Thr Val Met
1               5                   10                  15

<210>    109
<211>    15

<212>    PRT
<213>    Artificial Sequence
<220>
<223>    LMP2a epitope
<400>    109
Met Val Ala Gly Ala Val Trp Leu Thr Val Met Thr Asn Thr Leu
1               5                   10                  15

<210>    110
<211>    15
<212>    PRT
<213>    Artificial Sequence
<220>
<223>    LMP2a epitope
<400>    110
Ala Val Trp Leu Thr Val Met Thr Asn Thr Leu Leu Ser Ala Trp
1               5                   10                  15

<210>    111
<211>    15
<212>    PRT
<213>    Artificial Sequence
<220>
<223>    LMP2a epitope
<400>    111
Thr Val Met Thr Asn Thr Leu Leu Ser Ala Trp Ile Leu Thr Ala
1               5                   10                  15

<210>    112
<211>    15
<212>    PRT
<213>    Artificial Sequence
<220>
<223>    LMP2a epitope
<400>    112
Asn Thr Leu Leu Ser Ala Trp Ile Leu Thr Ala Gly Phe Leu Ile
1               5                   10                  15

<210>    113
<211>    15
<212>    PRT
<213>    Artificial Sequence
<220>
<223>    LMP2a epitope
<400>    113
Ser Ala Trp Ile Leu Thr Ala Gly Phe Leu Ile Phe Leu Ile Gly
1               5                   10                  15

<210>    114
<211>    15
<212>    PRT

<213>    Artificial Sequence
<220>
<223>    LMP2a epitope
<400>    114
Leu Thr Ala Gly Phe Leu Ile Phe Leu Ile Gly Phe Ala Leu Phe
1               5                   10                  15

<210>    115
<211>    15
<212>    PRT
<213>    Artificial Sequence
<220>
<223>    LMP2a epitope
<400>    115
Phe Leu Ile Phe Leu Ile Gly Phe Ala Leu Phe Gly Val Ile Arg
1               5                   10                  15

<210>    116
<211>    15
<212>    PRT
<213>    Artificial Sequence
<220>
<223>    LMP2a epitope
<400>    116
Leu Ile Gly Phe Ala Leu Phe Gly Val Ile Arg Cys Cys Arg Tyr
1               5                   10                  15

<210>    117
<211>    15
<212>    PRT
<213>    Artificial Sequence
<220>
<223>    LMP2a epitope
<400>    117
Ala Leu Phe Gly Val Ile Arg Cys Cys Arg Tyr Cys Cys Tyr Tyr
1               5                   10                  15

<210>    118
<211>    15
<212>    PRT
<213>    Artificial Sequence
<220>
<223>    LMP2a epitope
<400>    118
Val Ile Arg Cys Cys Arg Tyr Cys Cys Tyr Tyr Cys Leu Thr Leu
1               5                   10                  15

<210>    119
<211>    15
<212>    PRT
<213>    Artificial Sequence

<220>
<223>    LMP2a epitope
<400>    119
Cys Arg Tyr Cys Cys Tyr Tyr Cys Leu Thr Leu Glu Ser Glu Glu
1               5                   10                  15

<210>    120
<211>    15
<212>    PRT
<213>    Artificial Sequence
<220>
<223>    LMP2a epitope
<400>    120
Cys Tyr Tyr Cys Leu Thr Leu Glu Ser Glu Glu Arg Pro Pro Thr
1               5                   10                  15

<210>    121
<211>    15
<212>    PRT
<213>    Artificial Sequence
<220>
<223>    LMP2a epitope
<400>    121
Leu Thr Leu Glu Ser Glu Glu Arg Pro Pro Thr Pro Tyr Arg Asn
1               5                   10                  15

<210>    122
<211>    15
<212>    PRT
<213>    Artificial Sequence
<220>
<223>    LMP2a epitope
<400>    122
Leu Glu Ser Glu Glu Arg Pro Pro Thr Pro Tyr Arg Asn Thr Val
1               5                   10                  15

<210>    123
<211>    15
<212>    PRT
<213>    Artificial Sequence
<220>
<223>    EBNA-1 epitope
<400>    123
Gly Gly Ala Gly Ala Gly Gly Gly Gly Arg Gly Arg Gly Gly Ser
1               5                   10                  15

<210>    124
<211>    15
<212>    PRT
<213>    Artificial Sequence
<220>

<223>    EBNA-1 epitope
<400>    124
Ala Gly Gly Gly Gly Arg Gly Arg Gly Gly Ser Gly Gly Arg Gly
1               5                   10                  15

<210>    125
<211>    15
<212>    PRT
<213>    Artificial Sequence
<220>
<223>    EBNA-1 epitope
<400>    125
Gly Arg Gly Arg Gly Gly Ser Gly Gly Arg Gly Arg Gly Gly Ser
1               5                   10                  15

<210>    126
<211>    15
<212>    PRT
<213>    Artificial Sequence
<220>
<223>    EBNA-1 epitope
<400>    126
Gly Gly Ser Gly Gly Arg Gly Arg Gly Gly Ser Gly Gly Arg Gly
1               5                   10                  15

<210>    127
<211>    15
<212>    PRT
<213>    Artificial Sequence
<220>
<223>    EBNA-1 epitope
<400>    127
Gly Arg Gly Arg Gly Gly Ser Gly Gly Arg Gly Arg Gly Gly Ser
1               5                   10                  15

<210>    128
<211>    15
<212>    PRT
<213>    Artificial Sequence
<220>
<223>    EBNA-1 epitope
<400>    128
Gly Gly Ser Gly Gly Arg Gly Arg Gly Gly Ser Gly Gly Arg Arg
1               5                   10                  15

<210>    129
<211>    15
<212>    PRT
<213>    Artificial Sequence
<220>
<223>    EBNA-1 epitope

<400>    129
Gly Arg Gly Arg Gly Gly Ser Gly Gly Arg Arg Gly Arg Gly Arg
1               5                   10                  15

<210>    130
<211>    15
<212>    PRT
<213>    Artificial Sequence
<220>
<223>    EBNA-1 epitope
<400>    130
Gly Gly Ser Gly Gly Arg Arg Gly Arg Gly Arg Glu Arg Ala Arg
1               5                   10                  15

<210>    131
<211>    15
<212>    PRT
<213>    Artificial Sequence
<220>
<223>    EBNA-1 epitope
<400>    131
Gly Arg Arg Gly Arg Gly Arg Glu Arg Ala Arg Gly Gly Ser Arg
1               5                   10                  15

<210>    132
<211>    15
<212>    PRT
<213>    Artificial Sequence
<220>
<223>    EBNA-1 epitope
<400>    132
Arg Gly Arg Glu Arg Ala Arg Gly Gly Ser Arg Glu Arg Ala Arg
1               5                   10                  15

<210>    133
<211>    15
<212>    PRT
<213>    Artificial Sequence
<220>
<223>    EBNA-1 epitope
<400>    133
Arg Ala Arg Gly Gly Ser Arg Glu Arg Ala Arg Gly Arg Gly Arg
1               5                   10                  15

<210>    134
<211>    15
<212>    PRT
<213>    Artificial Sequence
<220>
<223>    EBNA-1 epitope
<400>    134

Gly Ser Arg Glu Arg Ala Arg Gly Arg Gly Arg Gly Arg Gly Glu
1               5                   10                  15

<210>    135
<211>    15
<212>    PRT
<213>    Artificial Sequence
<220>
<223>    EBNA-1 epitope
<400>    135
Arg Ala Arg Gly Arg Gly Arg Gly Arg Gly Glu Lys Arg Pro Arg
1               5                   10                  15

<210>    136
<211>    15
<212>    PRT
<213>    Artificial Sequence
<220>
<223>    EBNA-1 epitope
<400>    136
Arg Gly Arg Gly Arg Gly Glu Lys Arg Pro Arg Ser Pro Ser Ser
1               5                   10                  15

<210>    137
<211>    15
<212>    PRT
<213>    Artificial Sequence
<220>
<223>    EBNA-1 epitope
<400>    137
Arg Gly Glu Lys Arg Pro Arg Ser Pro Ser Ser Gln Ser Ser Ser
1               5                   10                  15

<210>    138
<211>    15
<212>    PRT
<213>    Artificial Sequence
<220>
<223>    EBNA-1 epitope
<400>    138
Arg Pro Arg Ser Pro Ser Ser Gln Ser Ser Ser Ser Gly Ser Pro
1               5                   10                  15

<210>    139
<211>    15
<212>    PRT
<213>    Artificial Sequence
<220>
<223>    EBNA-1 epitope
<400>    139
Pro Ser Ser Gln Ser Ser Ser Ser Gly Ser Pro Pro Arg Arg Pro

1               5                   10                  15

<210>   140
<211>   15
<212>   PRT
<213>   Artificial Sequence
<220>
<223>   EBNA-1 epitope
<400>   140
Ser Ser Ser Ser Gly Ser Pro Pro Arg Arg Pro Pro Pro Gly Arg
1               5                   10                  15

<210>   141
<211>   15
<212>   PRT
<213>   Artificial Sequence
<220>
<223>   EBNA-1 epitope
<400>   141
Gly Ser Pro Pro Arg Arg Pro Pro Pro Gly Arg Arg Pro Phe Phe
1               5                   10                  15

<210>   142
<211>   15
<212>   PRT
<213>   Artificial Sequence
<220>
<223>   EBNA-1 epitope
<400>   142
Arg Arg Pro Pro Pro Gly Arg Arg Pro Phe Phe His Pro Val Gly
1               5                   10                  15

<210>   143
<211>   15
<212>   PRT
<213>   Artificial Sequence
<220>
<223>   EBNA-1 epitope
<400>   143
Pro Gly Arg Arg Pro Phe Phe His Pro Val Gly Asp Ala Asp Tyr
1               5                   10                  15

<210>   144
<211>   15
<212>   PRT
<213>   Artificial Sequence
<220>
<223>   EBNA-1 epitope
<400>   144
Pro Phe Phe His Pro Val Gly Asp Ala Asp Tyr Phe Glu Tyr Leu
1               5                   10                  15

<210> 145
<211> 15
<212> PRT
<213> Artificial Sequence
<220>
<223> EBNA-1 epitope
<400> 145
Pro Val Gly Asp Ala Asp Tyr Phe Glu Tyr Leu Gln Glu Gly Gly
1               5                   10                  15

<210> 146
<211> 15
<212> PRT
<213> Artificial Sequence
<220>
<223> EBNA-1 epitope
<400> 146
Ala Asp Tyr Phe Glu Tyr Leu Gln Glu Gly Gly Pro Asp Gly Glu
1               5                   10                  15

<210> 147
<211> 15
<212> PRT
<213> Artificial Sequence
<220>
<223> EBNA-1 epitope
<400> 147
Glu Tyr Leu Gln Glu Gly Gly Pro Asp Gly Glu Pro Asp Val Pro
1               5                   10                  15

<210> 148
<211> 15
<212> PRT
<213> Artificial Sequence
<220>
<223> EBNA-1 epitope
<400> 148
Glu Gly Gly Pro Asp Gly Glu Pro Asp Val Pro Pro Gly Ala Ile
1               5                   10                  15

<210> 149
<211> 15
<212> PRT
<213> Artificial Sequence
<220>
<223> EBNA-1 epitope
<400> 149
Asp Gly Glu Pro Asp Val Pro Pro Gly Ala Ile Glu Gln Gly Pro
1               5                   10                  15

<210>	150
<211>	15
<212>	PRT
<213>	Artificial Sequence
<220>
<223>	EBNA-1 epitope
<400>	150
Asp Val Pro Pro Gly Ala Ile Glu Gln Gly Pro Thr Asp Asp Pro
1               5                   10                  15

<210>	151
<211>	15
<212>	PRT
<213>	Artificial Sequence
<220>
<223>	EBNA-1 epitope
<400>	151
Gly Ala Ile Glu Gln Gly Pro Thr Asp Asp Pro Gly Glu Gly Pro
1               5                   10                  15

<210>	152
<211>	15
<212>	PRT
<213>	Artificial Sequence
<220>
<223>	EBNA-1 epitope
<400>	152
Gln Gly Pro Thr Asp Asp Pro Gly Glu Gly Pro Ser Thr Gly Pro
1               5                   10                  15

<210>	153
<211>	15
<212>	PRT
<213>	Artificial Sequence
<220>
<223>	EBNA-1 epitope
<400>	153
Asp Asp Pro Gly Glu Gly Pro Ser Thr Gly Pro Arg Gly Gln Gly
1               5                   10                  15

<210>	154
<211>	15
<212>	PRT
<213>	Artificial Sequence
<220>
<223>	EBNA-1 epitope
<400>	154
Glu Gly Pro Ser Thr Gly Pro Arg Gly Gln Gly Asp Gly Gly Arg
1               5                   10                  15

<210>	155

<211>	15
<212>	PRT
<213>	Artificial Sequence
<220>
<223>	EBNA-1 epitope
<400>	155
Thr Gly Pro Arg Gly Gln Gly Asp Gly Gly Arg Arg Lys Lys Gly
1               5                   10                  15

<210>	156
<211>	15
<212>	PRT
<213>	Artificial Sequence
<220>
<223>	EBNA-1 epitope
<400>	156
Gly Gln Gly Asp Gly Gly Arg Arg Lys Lys Gly Gly Trp Phe Gly
1               5                   10                  15

<210>	157
<211>	15
<212>	PRT
<213>	Artificial Sequence
<220>
<223>	EBNA-1 epitope
<400>	157
Gly Gly Arg Arg Lys Lys Gly Gly Trp Phe Gly Lys His Arg Gly
1               5                   10                  15

<210>	158
<211>	15
<212>	PRT
<213>	Artificial Sequence
<220>
<223>	EBNA-1 epitope
<400>	158
Lys Lys Gly Gly Trp Phe Gly Lys His Arg Gly Gln Gly Gly Ser
1               5                   10                  15

<210>	159
<211>	15
<212>	PRT
<213>	Artificial Sequence
<220>
<223>	EBNA-1 epitope
<400>	159
Trp Phe Gly Lys His Arg Gly Gln Gly Gly Ser Asn Pro Lys Phe
1               5                   10                  15

<210>	160
<211>	15

<212>	PRT
<213>	Artificial Sequence
<220>
<223>	EBNA-1 epitope
<400>	160
His Arg Gly Gln Gly Gly Ser Asn Pro Lys Phe Glu Asn Ile Ala
1               5                   10                  15

<210>	161
<211>	15
<212>	PRT
<213>	Artificial Sequence
<220>
<223>	EBNA-1 epitope
<400>	161
Gly Gly Ser Asn Pro Lys Phe Glu Asn Ile Ala Glu Gly Leu Arg
1               5                   10                  15

<210>	162
<211>	15
<212>	PRT
<213>	Artificial Sequence
<220>
<223>	EBNA-1 epitope
<400>	162
Pro Lys Phe Glu Asn Ile Ala Glu Gly Leu Arg Val Leu Leu Ala
1               5                   10                  15

<210>	163
<211>	15
<212>	PRT
<213>	Artificial Sequence
<220>
<223>	EBNA-1 epitope
<400>	163
Asn Ile Ala Glu Gly Leu Arg Val Leu Leu Ala Arg Ser His Val
1               5                   10                  15

<210>	164
<211>	15
<212>	PRT
<213>	Artificial Sequence
<220>
<223>	EBNA-1 epitope
<400>	164
Gly Leu Arg Val Leu Leu Ala Arg Ser His Val Glu Arg Thr Thr
1               5                   10                  15

<210>	165
<211>	15
<212>	PRT

<213>    Artificial Sequence
<220>
<223>    EBNA-1 epitope
<400>    165
Leu Leu Ala Arg Ser His Val Glu Arg Thr Thr Glu Glu Gly Asn
1               5                   10                  15

<210>    166
<211>    15
<212>    PRT
<213>    Artificial Sequence
<220>
<223>    EBNA-1 epitope
<400>    166
Ser His Val Glu Arg Thr Thr Glu Glu Gly Asn Trp Val Ala Gly
1               5                   10                  15

<210>    167
<211>    15
<212>    PRT
<213>    Artificial Sequence
<220>
<223>    EBNA-1 epitope
<400>    167
Arg Thr Thr Glu Glu Gly Asn Trp Val Ala Gly Val Phe Val Tyr
1               5                   10                  15

<210>    168
<211>    15
<212>    PRT
<213>    Artificial Sequence
<220>
<223>    EBNA-1 epitope
<400>    168
Glu Gly Asn Trp Val Ala Gly Val Phe Val Tyr Gly Gly Ser Lys
1               5                   10                  15

<210>    169
<211>    15
<212>    PRT
<213>    Artificial Sequence
<220>
<223>    EBNA-1 epitope
<400>    169
Val Ala Gly Val Phe Val Tyr Gly Gly Ser Lys Thr Ser Leu Tyr
1               5                   10                  15

<210>    170
<211>    15
<212>    PRT
<213>    Artificial Sequence

<220>
<223>    EBNA-1 epitope
<400>    170
Phe Val Tyr Gly Gly Ser Lys Thr Ser Leu Tyr Asn Leu Arg Arg
1               5                   10                  15

<210>    171
<211>    15
<212>    PRT
<213>    Artificial Sequence
<220>
<223>    EBNA-1 epitope
<400>    171
Gly Ser Lys Thr Ser Leu Tyr Asn Leu Arg Arg Gly Ile Ala Leu
1               5                   10                  15

<210>    172
<211>    15
<212>    PRT
<213>    Artificial Sequence
<220>
<223>    EBNA-1 epitope
<400>    172
Ser Leu Tyr Asn Leu Arg Arg Gly Ile Ala Leu Ala Val Pro Gln
1               5                   10                  15

<210>    173
<211>    15
<212>    PRT
<213>    Artificial Sequence
<220>
<223>    EBNA-1 epitope
<400>    173
Leu Arg Arg Gly Ile Ala Leu Ala Val Pro Gln Cys Arg Ile Thr
1               5                   10                  15

<210>    174
<211>    15
<212>    PRT
<213>    Artificial Sequence
<220>
<223>    EBNA-1 epitope
<400>    174
Ile Ala Leu Ala Val Pro Gln Cys Arg Ile Thr Pro Leu Ser Arg
1               5                   10                  15

<210>    175
<211>    15
<212>    PRT
<213>    Artificial Sequence
<220>

<223>    EBNA-1 epitope
<400>    175
Val Pro Gln Cys Arg Ile Thr Pro Leu Ser Arg Leu Pro Phe Gly
1               5                   10                  15

<210>    176
<211>    15
<212>    PRT
<213>    Artificial Sequence
<220>
<223>    EBNA-1 epitope
<400>    176
Arg Ile Thr Pro Leu Ser Arg Leu Pro Phe Gly Met Ala Pro Gly
1               5                   10                  15

<210>    177
<211>    15
<212>    PRT
<213>    Artificial Sequence
<220>
<223>    EBNA-1 epitope
<400>    177
Leu Ser Arg Leu Pro Phe Gly Met Ala Pro Gly Pro Gly Pro Gln
1               5                   10                  15

<210>    178
<211>    15
<212>    PRT
<213>    Artificial Sequence
<220>
<223>    EBNA-1 epitope
<400>    178
Pro Phe Gly Met Ala Pro Gly Pro Gly Pro Gln Pro Gly Pro Leu
1               5                   10                  15

<210>    179
<211>    15
<212>    PRT
<213>    Artificial Sequence
<220>
<223>    EBNA-1 epitope
<400>    179
Ala Pro Gly Pro Gly Pro Gln Pro Gly Pro Leu Arg Glu Ser Ile
1               5                   10                  15

<210>    180
<211>    15
<212>    PRT
<213>    Artificial Sequence
<220>
<223>    EBNA-1 epitope

<400>    180
Gly Pro Gln Pro Gly Pro Leu Arg Glu Ser Ile Val Cys Tyr Phe
1               5                   10                  15

<210>    181
<211>    15
<212>    PRT
<213>    Artificial Sequence
<220>
<223>    EBNA-1 epitope
<400>    181
Gly Pro Leu Arg Glu Ser Ile Val Cys Tyr Phe Met Val Phe Leu
1               5                   10                  15

<210>    182
<211>    15
<212>    PRT
<213>    Artificial Sequence
<220>
<223>    EBNA-1 epitope
<400>    182
Glu Ser Ile Val Cys Tyr Phe Met Val Phe Leu Gln Thr His Ile
1               5                   10                  15

<210>    183
<211>    15
<212>    PRT
<213>    Artificial Sequence
<220>
<223>    EBNA-1 epitope
<400>    183
Cys Tyr Phe Met Val Phe Leu Gln Thr His Ile Phe Ala Glu Val
1               5                   10                  15

<210>    184
<211>    15
<212>    PRT
<213>    Artificial Sequence
<220>
<223>    EBNA-1 epitope
<400>    184
Val Phe Leu Gln Thr His Ile Phe Ala Glu Val Leu Lys Asp Ala
1               5                   10                  15

<210>    185
<211>    15
<212>    PRT
<213>    Artificial Sequence
<220>
<223>    EBNA-1 epitope
<400>    185

Thr His Ile Phe Ala Glu Val Leu Lys Asp Ala Ile Lys Asp Leu
1               5                   10                  15

<210>   186
<211>   15
<212>   PRT
<213>   Artificial Sequence
<220>
<223>   EBNA-1 epitope
<400>   186
Ala Glu Val Leu Lys Asp Ala Ile Lys Asp Leu Val Met Thr Lys
1               5                   10                  15

<210>   187
<211>   15
<212>   PRT
<213>   Artificial Sequence
<220>
<223>   EBNA-1 epitope
<400>   187
Lys Asp Ala Ile Lys Asp Leu Val Met Thr Lys Pro Ala Pro Thr
1               5                   10                  15

<210>   188
<211>   15
<212>   PRT
<213>   Artificial Sequence
<220>
<223>   EBNA-1 epitope
<400>   188
Lys Asp Leu Val Met Thr Lys Pro Ala Pro Thr Cys Asn Ile Lys
1               5                   10                  15

<210>   189
<211>   15
<212>   PRT
<213>   Artificial Sequence
<220>
<223>   EBNA-1 epitope
<400>   189
Met Thr Lys Pro Ala Pro Thr Cys Asn Ile Lys Val Thr Val Cys
1               5                   10                  15

<210>   190
<211>   15
<212>   PRT
<213>   Artificial Sequence
<220>
<223>   EBNA-1 epitope
<400>   190
Ala Pro Thr Cys Asn Ile Lys Val Thr Val Cys Ser Phe Asp Asp

1                   5                   10                  15

<210>    191
<211>    15
<212>    PRT
<213>    Artificial Sequence
<220>
<223>    EBNA-1 epitope
<400>    191
Asn Ile Lys Val Thr Val Cys Ser Phe Asp Asp Gly Val Asp Leu
1                   5                   10                  15

<210>    192
<211>    15
<212>    PRT
<213>    Artificial Sequence
<220>
<223>    EBNA-1 epitope
<400>    192
Thr Val Cys Ser Phe Asp Asp Gly Val Asp Leu Pro Pro Trp Phe
1                   5                   10                  15

<210>    193
<211>    15
<212>    PRT
<213>    Artificial Sequence
<220>
<223>    EBNA-1 epitope
<400>    193
Phe Asp Asp Gly Val Asp Leu Pro Pro Trp Phe Pro Pro Met Val
1                   5                   10                  15

<210>    194
<211>    15
<212>    PRT
<213>    Artificial Sequence
<220>
<223>    EBNA-1 epitope
<400>    194
Val Asp Leu Pro Pro Trp Phe Pro Pro Met Val Glu Gly Ala Ala
1                   5                   10                  15

<210>    195
<211>    15
<212>    PRT
<213>    Artificial Sequence
<220>
<223>    EBNA-1 epitope
<400>    195
Pro Trp Phe Pro Pro Met Val Glu Gly Ala Ala Ala Glu Gly Asp
1                   5                   10                  15

<210> 196
<211> 15
<212> PRT
<213> Artificial Sequence
<220>
<223> EBNA-1 epitope
<400> 196
Pro Met Val Glu Gly Ala Ala Ala Glu Gly Asp Asp Gly Asp Asp
1               5                   10                  15

<210> 197
<211> 15
<212> PRT
<213> Artificial Sequence
<220>
<223> EBNA-1 epitope
<400> 197
Gly Ala Ala Ala Glu Gly Asp Asp Gly Asp Asp Gly Asp Glu Gly
1               5                   10                  15

<210> 198
<211> 15
<212> PRT
<213> Artificial Sequence
<220>
<223> EBNA-1 epitope
<400> 198
Glu Gly Asp Asp Gly Asp Asp Gly Asp Glu Gly Gly Asp Gly Asp
1               5                   10                  15

<210> 199
<211> 15
<212> PRT
<213> Artificial Sequence
<220>
<223> EBNA-1 epitope
<400> 199
Gly Asp Asp Gly Asp Glu Gly Gly Asp Gly Asp Glu Gly Glu Glu
1               5                   10                  15

<210> 200
<211> 15
<212> PRT
<213> Artificial Sequence
<220>
<223> EBNA-1 epitope
<400> 200
Gly Asp Glu Gly Gly Asp Gly Asp Glu Gly Glu Glu Gly Gln Glu
1               5                   10                  15

<110>     Good T Cells, Inc.

<120>     A Method for activating T cells to treat cancer

<130>     POPB201876PCT

<150>     KR 10-2019-0015108
<151>     2019-02-08

<160>     200

<170>     KoPatentIn 3.0

<210>     1
<211>     15
<212>     PRT
<213>     Artificial Sequence

<220>
<223>     LMP2a epitope


<400>     1
Met Gly Ser Leu Glu Met Val Pro Met Gly Ala Gly Pro Pro Ser
  1               5                  10                  15


<210>     2
<211>     15
<212>     PRT
<213>     Artificial Sequence

<220>
<223>     LMP2a epitope


<400>     2
Glu Met Val Pro Met Gly Ala Gly Pro Pro Ser Pro Gly Gly Asp
  1               5                  10                  15


<210>     3
<211>     15
<212>     PRT
<213>     Artificial Sequence

<220>
<223>     LMP2a epitope


<400>     3
Met Gly Ala Gly Pro Pro Ser Pro Gly Gly Asp Pro Asp Gly Asp
  1               5                  10                  15


<210>     4
<211>     15
<212>     PRT
<213>     Artificial Sequence

<220>
<223>     LMP2a epitope

<400>    4
Pro Pro Ser Pro Gly Gly Asp Pro Asp Gly Asp Asp Gly Gly Asn
1               5                   10                  15

<210>    5
<211>    15
<212>    PRT
<213>    Artificial Sequence

<220>
<223>    LMP2a epitope

<400>    5
Gly Gly Asp Pro Asp Gly Asp Asp Gly Gly Asn Asn Ser Gln Tyr
1               5                   10                  15

<210>    6
<211>    15
<212>    PRT
<213>    Artificial Sequence

<220>
<223>    LMP2a epitope

<400>    6
Asp Gly Asp Asp Gly Gly Asn Asn Ser Gln Tyr Pro Ser Ala Ser
1               5                   10                  15

<210>    7
<211>    15
<212>    PRT
<213>    Artificial Sequence

<220>
<223>    LMP2a epitope

<400>    7
Gly Gly Asn Asn Ser Gln Tyr Pro Ser Ala Ser Gly Ser Tyr Gly
1               5                   10                  15

<210>    8
<211>    15
<212>    PRT
<213>    Artificial Sequence

<220>
<223>    LMP2a epitope

<400>    8
Ser Gln Tyr Pro Ser Ala Ser Gly Ser Tyr Gly Asn Thr Pro Thr
1               5                   10                  15

<210>    9

<211>    15
<212>    PRT
<213>    Artificial Sequence

<220>
<223>    LMP2a epitope


<400>    9
Ser Ala Ser Gly Ser Tyr Gly Asn Thr Pro Thr Pro Pro Asn Asp
1               5                   10                  15


<210>    10
<211>    15
<212>    PRT
<213>    Artificial Sequence

<220>
<223>    LMP2a epitope


<400>    10
Ser Tyr Gly Asn Thr Pro Thr Pro Pro Asn Asp Glu Glu Arg Glu
1               5                   10                  15


<210>    11
<211>    15
<212>    PRT
<213>    Artificial Sequence

<220>
<223>    LMP2a epitope


<400>    11
Thr Pro Thr Pro Pro Asn Asp Glu Glu Arg Glu Ser Asn Glu Glu
1               5                   10                  15


<210>    12
<211>    15
<212>    PRT
<213>    Artificial Sequence

<220>
<223>    LMP2a epitope


<400>    12
Pro Asn Asp Glu Glu Arg Glu Ser Asn Glu Glu Pro Pro Pro Pro
1               5                   10                  15


<210>    13
<211>    15
<212>    PRT
<213>    Artificial Sequence

<220>
<223>    LMP2a epitope

<400>    13
Glu Arg Glu Ser Asn Glu Glu Pro Pro Pro Pro Tyr Glu Asp Leu
1               5                   10                  15

<210>    14
<211>    15
<212>    PRT
<213>    Artificial Sequence

<220>
<223>    LMP2a epitope

<400>    14
Asn Glu Glu Pro Pro Pro Pro Tyr Glu Asp Leu Asp Trp Gly Asn
1               5                   10                  15

<210>    15
<211>    15
<212>    PRT
<213>    Artificial Sequence

<220>
<223>    LMP2a epitope

<400>    15
Pro Pro Pro Tyr Glu Asp Leu Asp Trp Gly Asn Gly Asp Arg His
1               5                   10                  15

<210>    16
<211>    15
<212>    PRT
<213>    Artificial Sequence

<220>
<223>    LMP2a epitope

<400>    16
Glu Asp Leu Asp Trp Gly Asn Gly Asp Arg His Ser Asp Tyr Gln
1               5                   10                  15

<210>    17
<211>    15
<212>    PRT
<213>    Artificial Sequence

<220>
<223>    LMP2a epitope

<400>    17
Trp Gly Asn Gly Asp Arg His Ser Asp Tyr Gln Pro Leu Gly Asn
1               5                   10                  15

<210>    18

<211>    15
<212>    PRT
<213>    Artificial Sequence

<220>
<223>    LMP2a epitope


<400>    18
Asp Arg His Ser Asp Tyr Gln Pro Leu Gly Asn Gln Asp Pro Ser
1               5                   10                  15


<210>    19
<211>    15
<212>    PRT
<213>    Artificial Sequence

<220>
<223>    LMP2a epitope


<400>    19
Asp Tyr Gln Pro Leu Gly Asn Gln Asp Pro Ser Leu Tyr Leu Gly
1               5                   10                  15


<210>    20
<211>    15
<212>    PRT
<213>    Artificial Sequence

<220>
<223>    LMP2a epitope


<400>    20
Leu Gly Asn Gln Asp Pro Ser Leu Tyr Leu Gly Leu Gln His Asp
1               5                   10                  15


<210>    21
<211>    15
<212>    PRT
<213>    Artificial Sequence

<220>
<223>    LMP2a epitope


<400>    21
Asp Pro Ser Leu Tyr Leu Gly Leu Gln His Asp Gly Asn Asp Gly
1               5                   10                  15


<210>    22
<211>    15
<212>    PRT
<213>    Artificial Sequence

<220>
<223>    LMP2a epitope

<400>    22
Tyr Leu Gly Leu Gln His Asp Gly Asn Asp Gly Leu Pro Pro Pro
1               5                   10                  15

<210>    23
<211>    15
<212>    PRT
<213>    Artificial Sequence

<220>
<223>    LMP2a epitope

<400>    23
Gln His Asp Gly Asn Asp Gly Leu Pro Pro Pro Pro Tyr Ser Pro
1               5                   10                  15

<210>    24
<211>    15
<212>    PRT
<213>    Artificial Sequence

<220>
<223>    LMP2a epitope

<400>    24
Asn Asp Gly Leu Pro Pro Pro Pro Tyr Ser Pro Arg Asp Asp Ser
1               5                   10                  15

<210>    25
<211>    15
<212>    PRT
<213>    Artificial Sequence

<220>
<223>    LMP2a epitope

<400>    25
Pro Pro Pro Pro Tyr Ser Pro Arg Asp Asp Ser Ser Gln His Ile
1               5                   10                  15

<210>    26
<211>    15
<212>    PRT
<213>    Artificial Sequence

<220>
<223>    LMP2a epitope

<400>    26
Tyr Ser Pro Arg Asp Asp Ser Ser Gln His Ile Tyr Glu Glu Ala
1               5                   10                  15

<210>    27

<211>    15
<212>    PRT
<213>    Artificial Sequence

<220>
<223>    LMP2a epitope


<400>    27
Asp Asp Ser Ser Gln His Ile Tyr Glu Glu Ala Gly Arg Gly Ser
1               5                   10                  15


<210>    28
<211>    15
<212>    PRT
<213>    Artificial Sequence

<220>
<223>    LMP2a epitope


<400>    28
Gln His Ile Tyr Glu Glu Ala Gly Arg Gly Ser Met Asn Pro Val
1               5                   10                  15


<210>    29
<211>    15
<212>    PRT
<213>    Artificial Sequence

<220>
<223>    LMP2a epitope


<400>    29
Glu Glu Ala Gly Arg Gly Ser Met Asn Pro Val Cys Leu Pro Val
1               5                   10                  15


<210>    30
<211>    15
<212>    PRT
<213>    Artificial Sequence

<220>
<223>    LMP2a epitope


<400>    30
Arg Gly Ser Met Asn Pro Val Cys Leu Pro Val Ile Val Ala Pro
1               5                   10                  15


<210>    31
<211>    15
<212>    PRT
<213>    Artificial Sequence

<220>
<223>    LMP2a epitope

<400>    31
Asn Pro Val Cys Leu Pro Val Ile Val Ala Pro Tyr Leu Phe Trp
1               5                   10                  15

<210>    32
<211>    15
<212>    PRT
<213>    Artificial Sequence

<220>
<223>    LMP2a epitope

<400>    32
Leu Pro Val Ile Val Ala Pro Tyr Leu Phe Trp Leu Ala Ala Ile
1               5                   10                  15

<210>    33
<211>    15
<212>    PRT
<213>    Artificial Sequence

<220>
<223>    LMP2a epitope

<400>    33
Val Ala Pro Tyr Leu Phe Trp Leu Ala Ala Ile Ala Ala Ser Cys
1               5                   10                  15

<210>    34
<211>    15
<212>    PRT
<213>    Artificial Sequence

<220>
<223>    LMP2a epitope

<400>    34
Leu Phe Trp Leu Ala Ala Ile Ala Ala Ser Cys Phe Thr Ala Ser
1               5                   10                  15

<210>    35
<211>    15
<212>    PRT
<213>    Artificial Sequence

<220>
<223>    LMP2a epitope

<400>    35
Ala Ala Ile Ala Ala Ser Cys Phe Thr Ala Ser Val Ser Thr Val
1               5                   10                  15

<210>    36

<211>    15
<212>    PRT
<213>    Artificial Sequence

<220>
<223>    LMP2a epitope


<400>    36
Ala Ser Cys Phe Thr Ala Ser Val Ser Thr Val Val Thr Ala Thr
1               5                   10                  15


<210>    37
<211>    15
<212>    PRT
<213>    Artificial Sequence

<220>
<223>    LMP2a epitope


<400>    37
Thr Ala Ser Val Ser Thr Val Val Thr Ala Thr Gly Leu Ala Leu
1               5                   10                  15


<210>    38
<211>    15
<212>    PRT
<213>    Artificial Sequence

<220>
<223>    LMP2a epitope


<400>    38
Ser Thr Val Val Thr Ala Thr Gly Leu Ala Leu Ser Leu Leu Leu
1               5                   10                  15


<210>    39
<211>    15
<212>    PRT
<213>    Artificial Sequence

<220>
<223>    LMP2a epitope


<400>    39
Thr Ala Thr Gly Leu Ala Leu Ser Leu Leu Leu Leu Ala Ala Val
1               5                   10                  15


<210>    40
<211>    15
<212>    PRT
<213>    Artificial Sequence

<220>
<223>    LMP2a epitope

<400>    40
Leu Ala Leu Ser Leu Leu Leu Leu Ala Ala Val Ala Ser Ser Tyr
1               5                   10                  15

<210>    41
<211>    15
<212>    PRT
<213>    Artificial Sequence

<220>
<223>    LMP2a epitope

<400>    41
Leu Leu Leu Leu Ala Ala Val Ala Ser Ser Tyr Ala Ala Ala Gln
1               5                   10                  15

<210>    42
<211>    15
<212>    PRT
<213>    Artificial Sequence

<220>
<223>    LMP2a epitope

<400>    42
Ala Ala Val Ala Ser Ser Tyr Ala Ala Ala Gln Arg Lys Leu Leu
1               5                   10                  15

<210>    43
<211>    15
<212>    PRT
<213>    Artificial Sequence

<220>
<223>    LMP2a epitope

<400>    43
Ser Ser Tyr Ala Ala Ala Gln Arg Lys Leu Leu Thr Pro Val Thr
1               5                   10                  15

<210>    44
<211>    15
<212>    PRT
<213>    Artificial Sequence

<220>
<223>    LMP2a epitope

<400>    44
Ala Ala Gln Arg Lys Leu Leu Thr Pro Val Thr Val Leu Thr Ala
1               5                   10                  15

<210>    45

<211>    15
<212>    PRT
<213>    Artificial Sequence

<220>
<223>    LMP2a epitope


<400>    45
Lys Leu Leu Thr Pro Val Thr Val Leu Thr Ala Val Val Thr Phe
1               5                   10                  15


<210>    46
<211>    15
<212>    PRT
<213>    Artificial Sequence

<220>
<223>    LMP2a epitope


<400>    46
Pro Val Thr Val Leu Thr Ala Val Val Thr Phe Phe Ala Ile Cys
1               5                   10                  15


<210>    47
<211>    15
<212>    PRT
<213>    Artificial Sequence

<220>
<223>    LMP2a epitope


<400>    47
Leu Thr Ala Val Val Thr Phe Phe Ala Ile Cys Leu Thr Trp Arg
1               5                   10                  15


<210>    48
<211>    15
<212>    PRT
<213>    Artificial Sequence

<220>
<223>    LMP2a epitope


<400>    48
Val Thr Phe Phe Ala Ile Cys Leu Thr Trp Arg Ile Glu Asp Pro
1               5                   10                  15


<210>    49
<211>    15
<212>    PRT
<213>    Artificial Sequence

<220>
<223>    LMP2a epitope

<400>    49
Ala Ile Cys Leu Thr Trp Arg Ile Glu Asp Pro Pro Phe Asn Ser
1               5                   10                  15

<210>    50
<211>    15
<212>    PRT
<213>    Artificial Sequence

<220>
<223>    LMP2a epitope

<400>    50
Thr Trp Arg Ile Glu Asp Pro Pro Phe Asn Ser Leu Leu Phe Ala
1               5                   10                  15

<210>    51
<211>    15
<212>    PRT
<213>    Artificial Sequence

<220>
<223>    LMP2a epitope

<400>    51
Glu Asp Pro Pro Phe Asn Ser Leu Leu Phe Ala Leu Leu Ala Ala
1               5                   10                  15

<210>    52
<211>    15
<212>    PRT
<213>    Artificial Sequence

<220>
<223>    LMP2a epitope

<400>    52
Phe Asn Ser Leu Leu Phe Ala Leu Leu Ala Ala Ala Gly Gly Leu
1               5                   10                  15

<210>    53
<211>    15
<212>    PRT
<213>    Artificial Sequence

<220>
<223>    LMP2a epitope

<400>    53
Leu Phe Ala Leu Leu Ala Ala Ala Gly Gly Leu Gln Gly Ile Tyr
1               5                   10                  15

<210>    54

<211>    15
<212>    PRT
<213>    Artificial Sequence

<220>
<223>    LMP2a epitope


<400>    54
Leu Ala Ala Ala Gly Gly Leu Gln Gly Ile Tyr Val Leu Val Met
1               5                   10                  15


<210>    55
<211>    15
<212>    PRT
<213>    Artificial Sequence

<220>
<223>    LMP2a epitope


<400>    55
Gly Gly Leu Gln Gly Ile Tyr Val Leu Val Met Leu Val Leu Leu
1               5                   10                  15


<210>    56
<211>    15
<212>    PRT
<213>    Artificial Sequence

<220>
<223>    LMP2a epitope


<400>    56
Gly Ile Tyr Val Leu Val Met Leu Val Leu Leu Ile Leu Ala Tyr
1               5                   10                  15


<210>    57
<211>    15
<212>    PRT
<213>    Artificial Sequence

<220>
<223>    LMP2a epitope


<400>    57
Leu Val Met Leu Val Leu Leu Ile Leu Ala Tyr Arg Arg Arg Trp
1               5                   10                  15


<210>    58
<211>    15
<212>    PRT
<213>    Artificial Sequence

<220>
<223>    LMP2a epitope

<400>    58
Val Leu Leu Ile Leu Ala Tyr Arg Arg Arg Trp Arg Arg Leu Thr
1               5                   10                  15

<210>    59
<211>    15
<212>    PRT
<213>    Artificial Sequence

<220>
<223>    LMP2a epitope

<400>    59
Leu Ala Tyr Arg Arg Arg Trp Arg Arg Leu Thr Val Cys Gly Gly
1               5                   10                  15

<210>    60
<211>    15
<212>    PRT
<213>    Artificial Sequence

<220>
<223>    LMP2a epitope

<400>    60
Arg Arg Trp Arg Arg Leu Thr Val Cys Gly Gly Ile Met Phe Leu
1               5                   10                  15

<210>    61
<211>    15
<212>    PRT
<213>    Artificial Sequence

<220>
<223>    LMP2a epitope

<400>    61
Arg Leu Thr Val Cys Gly Gly Ile Met Phe Leu Ala Cys Val Leu
1               5                   10                  15

<210>    62
<211>    15
<212>    PRT
<213>    Artificial Sequence

<220>
<223>    LMP2a epitope

<400>    62
Cys Gly Gly Ile Met Phe Leu Ala Cys Val Leu Val Leu Ile Val
1               5                   10                  15

<210>    63

<211>    15
<212>    PRT
<213>    Artificial Sequence

<220>
<223>    LMP2a epitope


<400>    63
Met Phe Leu Ala Cys Val Leu Val Leu Ile Val Asp Ala Val Leu
1               5                   10                  15


<210>    64
<211>    15
<212>    PRT
<213>    Artificial Sequence

<220>
<223>    LMP2a epitope


<400>    64
Cys Val Leu Val Leu Ile Val Asp Ala Val Leu Gln Leu Ser Pro
1               5                   10                  15


<210>    65
<211>    15
<212>    PRT
<213>    Artificial Sequence

<220>
<223>    LMP2a epitope


<400>    65
Leu Ile Val Asp Ala Val Leu Gln Leu Ser Pro Leu Leu Gly Ala
1               5                   10                  15


<210>    66
<211>    15
<212>    PRT
<213>    Artificial Sequence

<220>
<223>    LMP2a epitope


<400>    66
Ala Val Leu Gln Leu Ser Pro Leu Leu Gly Ala Val Thr Val Val
1               5                   10                  15


<210>    67
<211>    15
<212>    PRT
<213>    Artificial Sequence

<220>
<223>    LMP2a epitope

<400>    67
Leu Ser Pro Leu Leu Gly Ala Val Thr Val Val Ser Met Thr Leu
1               5                   10                  15

<210>    68
<211>    15
<212>    PRT
<213>    Artificial Sequence

<220>
<223>    LMP2a epitope

<400>    68
Leu Gly Ala Val Thr Val Val Ser Met Thr Leu Leu Leu Leu Ala
1               5                   10                  15

<210>    69
<211>    15
<212>    PRT
<213>    Artificial Sequence

<220>
<223>    LMP2a epitope

<400>    69
Thr Val Val Ser Met Thr Leu Leu Leu Leu Ala Phe Val Leu Trp
1               5                   10                  15

<210>    70
<211>    15
<212>    PRT
<213>    Artificial Sequence

<220>
<223>    LMP2a epitope

<400>    70
Met Thr Leu Leu Leu Leu Ala Phe Val Leu Trp Leu Ser Ser Pro
1               5                   10                  15

<210>    71
<211>    15
<212>    PRT
<213>    Artificial Sequence

<220>
<223>    LMP2a epitope

<400>    71
Leu Leu Ala Phe Val Leu Trp Leu Ser Ser Pro Gly Gly Leu Gly
1               5                   10                  15

<210>    72

<211>    15
<212>    PRT
<213>    Artificial Sequence

<220>
<223>    LMP2a epitope


<400>    72
Val Leu Trp Leu Ser Ser Pro Gly Gly Leu Gly Thr Leu Gly Ala
1               5                   10                  15


<210>    73
<211>    15
<212>    PRT
<213>    Artificial Sequence

<220>
<223>    LMP2a epitope


<400>    73
Ser Ser Pro Gly Gly Leu Gly Thr Leu Gly Ala Ala Leu Leu Thr
1               5                   10                  15


<210>    74
<211>    15
<212>    PRT
<213>    Artificial Sequence

<220>
<223>    LMP2a epitope


<400>    74
Gly Leu Gly Thr Leu Gly Ala Ala Leu Leu Thr Leu Ala Ala Ala
1               5                   10                  15


<210>    75
<211>    15
<212>    PRT
<213>    Artificial Sequence

<220>
<223>    LMP2a epitope


<400>    75
Leu Gly Ala Ala Leu Leu Thr Leu Ala Ala Ala Leu Ala Leu Leu
1               5                   10                  15


<210>    76
<211>    15
<212>    PRT
<213>    Artificial Sequence

<220>
<223>    LMP2a epitope

<400>    76
Leu Leu Thr Leu Ala Ala Ala Leu Ala Leu Leu Ala Ser Leu Ile
1               5                   10                  15

<210>    77
<211>    15
<212>    PRT
<213>    Artificial Sequence

<220>
<223>    LMP2a epitope

<400>    77
Ala Ala Ala Leu Ala Leu Leu Ala Ser Leu Ile Leu Gly Thr Leu
1               5                   10                  15

<210>    78
<211>    15
<212>    PRT
<213>    Artificial Sequence

<220>
<223>    LMP2a epitope

<400>    78
Ala Leu Leu Ala Ser Leu Ile Leu Gly Thr Leu Asn Leu Thr Thr
1               5                   10                  15

<210>    79
<211>    15
<212>    PRT
<213>    Artificial Sequence

<220>
<223>    LMP2a epitope

<400>    79
Ser Leu Ile Leu Gly Thr Leu Asn Leu Thr Thr Met Phe Leu Leu
1               5                   10                  15

<210>    80
<211>    15
<212>    PRT
<213>    Artificial Sequence

<220>
<223>    LMP2a epitope

<400>    80
Gly Thr Leu Asn Leu Thr Thr Met Phe Leu Leu Met Leu Leu Trp
1               5                   10                  15

<210>    81

<211>    15
<212>    PRT
<213>    Artificial Sequence

<220>
<223>    LMP2a epitope


<400>    81
Leu Thr Thr Met Phe Leu Leu Met Leu Leu Trp Thr Leu Val Val
1               5                   10                  15


<210>    82
<211>    15
<212>    PRT
<213>    Artificial Sequence

<220>
<223>    LMP2a epitope


<400>    82
Phe Leu Leu Met Leu Leu Trp Thr Leu Val Val Leu Leu Ile Cys
1               5                   10                  15


<210>    83
<211>    15
<212>    PRT
<213>    Artificial Sequence

<220>
<223>    LMP2a epitope


<400>    83
Leu Leu Trp Thr Leu Val Val Leu Leu Ile Cys Ser Ser Cys Ser
1               5                   10                  15


<210>    84
<211>    15
<212>    PRT
<213>    Artificial Sequence

<220>
<223>    LMP2a epitope


<400>    84
Leu Val Val Leu Leu Ile Cys Ser Ser Cys Ser Ser Cys Pro Leu
1               5                   10                  15


<210>    85
<211>    15
<212>    PRT
<213>    Artificial Sequence

<220>
<223>    LMP2a epitope

<400>    85
Leu Ile Cys Ser Ser Cys Ser Ser Cys Pro Leu Thr Lys Ile Leu
1               5                   10                  15

<210>    86
<211>    15
<212>    PRT
<213>    Artificial Sequence

<220>
<223>    LMP2a epitope

<400>    86
Ser Cys Ser Ser Cys Pro Leu Thr Lys Ile Leu Leu Ala Arg Leu
1               5                   10                  15

<210>    87
<211>    15
<212>    PRT
<213>    Artificial Sequence

<220>
<223>    LMP2a epitope

<400>    87
Cys Pro Leu Thr Lys Ile Leu Leu Ala Arg Leu Phe Leu Tyr Ala
1               5                   10                  15

<210>    88
<211>    15
<212>    PRT
<213>    Artificial Sequence

<220>
<223>    LMP2a epitope

<400>    88
Lys Ile Leu Leu Ala Arg Leu Phe Leu Tyr Ala Leu Ala Leu Leu
1               5                   10                  15

<210>    89
<211>    15
<212>    PRT
<213>    Artificial Sequence

<220>
<223>    LMP2a epitope

<400>    89
Ala Arg Leu Phe Leu Tyr Ala Leu Ala Leu Leu Leu Leu Ala Ser
1               5                   10                  15

<210>    90

<211>    15
<212>    PRT
<213>    Artificial Sequence

<220>
<223>    LMP2a epitope


<400>    90
Leu Tyr Ala Leu Ala Leu Leu Leu Leu Ala Ser Ala Leu Ile Ala
1               5                   10                  15


<210>    91
<211>    15
<212>    PRT
<213>    Artificial Sequence

<220>
<223>    LMP2a epitope


<400>    91
Ala Leu Leu Leu Leu Ala Ser Ala Leu Ile Ala Gly Gly Ser Ile
1               5                   10                  15


<210>    92
<211>    15
<212>    PRT
<213>    Artificial Sequence

<220>
<223>    LMP2a epitope


<400>    92
Leu Ala Ser Ala Leu Ile Ala Gly Gly Ser Ile Leu Gln Thr Asn
1               5                   10                  15


<210>    93
<211>    15
<212>    PRT
<213>    Artificial Sequence

<220>
<223>    LMP2a epitope


<400>    93
Leu Ile Ala Gly Gly Ser Ile Leu Gln Thr Asn Phe Lys Ser Leu
1               5                   10                  15


<210>    94
<211>    15
<212>    PRT
<213>    Artificial Sequence

<220>
<223>    LMP2a epitope

<400>    94
Gly Ser Ile Leu Gln Thr Asn Phe Lys Ser Leu Ser Ser Thr Glu
1               5                   10                  15

<210>    95
<211>    15
<212>    PRT
<213>    Artificial Sequence

<220>
<223>    LMP2a epitope

<400>    95
Gln Thr Asn Phe Lys Ser Leu Ser Ser Thr Glu Phe Ile Pro Asn
1               5                   10                  15

<210>    96
<211>    15
<212>    PRT
<213>    Artificial Sequence

<220>
<223>    LMP2a epitope

<400>    96
Lys Ser Leu Ser Ser Thr Glu Phe Ile Pro Asn Leu Phe Cys Met
1               5                   10                  15

<210>    97
<211>    15
<212>    PRT
<213>    Artificial Sequence

<220>
<223>    LMP2a epitope

<400>    97
Ser Thr Glu Phe Ile Pro Asn Leu Phe Cys Met Leu Leu Leu Ile
1               5                   10                  15

<210>    98
<211>    15
<212>    PRT
<213>    Artificial Sequence

<220>
<223>    LMP2a epitope

<400>    98
Ile Pro Asn Leu Phe Cys Met Leu Leu Leu Ile Val Ala Gly Ile
1               5                   10                  15

<210>    99

<211>     15
<212>     PRT
<213>     Artificial Sequence

<220>
<223>     LMP2a epitope


<400>     99
Phe Cys Met Leu Leu Leu Ile Val Ala Gly Ile Leu Phe Ile Leu
1               5                   10                  15


<210>     100
<211>     15
<212>     PRT
<213>     Artificial Sequence

<220>
<223>     LMP2a epitope


<400>     100
Leu Leu Ile Val Ala Gly Ile Leu Phe Ile Leu Ala Ile Leu Thr
1               5                   10                  15


<210>     101
<211>     15
<212>     PRT
<213>     Artificial Sequence

<220>
<223>     LMP2a epitope


<400>     101
Ala Gly Ile Leu Phe Ile Leu Ala Ile Leu Thr Glu Trp Gly Ser
1               5                   10                  15


<210>     102
<211>     15
<212>     PRT
<213>     Artificial Sequence

<220>
<223>     LMP2a epitope


<400>     102
Phe Ile Leu Ala Ile Leu Thr Glu Trp Gly Ser Gly Asn Arg Thr
1               5                   10                  15


<210>     103
<211>     15
<212>     PRT
<213>     Artificial Sequence

<220>
<223>     LMP2a epitope

<400>    103
Ile Leu Thr Glu Trp Gly Ser Gly Asn Arg Thr Tyr Gly Pro Val
1               5                   10                  15

<210>    104
<211>    15
<212>    PRT
<213>    Artificial Sequence

<220>
<223>    LMP2a epitope

<400>    104
Trp Gly Ser Gly Asn Arg Thr Tyr Gly Pro Val Phe Met Cys Leu
1               5                   10                  15

<210>    105
<211>    15
<212>    PRT
<213>    Artificial Sequence

<220>
<223>    LMP2a epitope

<400>    105
Asn Arg Thr Tyr Gly Pro Val Phe Met Cys Leu Gly Gly Leu Leu
1               5                   10                  15

<210>    106
<211>    15
<212>    PRT
<213>    Artificial Sequence

<220>
<223>    LMP2a epitope

<400>    106
Gly Pro Val Phe Met Cys Leu Gly Gly Leu Leu Thr Met Val Ala
1               5                   10                  15

<210>    107
<211>    15
<212>    PRT
<213>    Artificial Sequence

<220>
<223>    LMP2a epitope

<400>    107
Met Cys Leu Gly Gly Leu Leu Thr Met Val Ala Gly Ala Val Trp
1               5                   10                  15

<210>    108

<211>    15
<212>    PRT
<213>    Artificial Sequence

<220>
<223>    LMP2a epitope


<400>    108
Gly Leu Leu Thr Met Val Ala Gly Ala Val Trp Leu Thr Val Met
1               5                   10                  15


<210>    109
<211>    15
<212>    PRT
<213>    Artificial Sequence

<220>
<223>    LMP2a epitope


<400>    109
Met Val Ala Gly Ala Val Trp Leu Thr Val Met Thr Asn Thr Leu
1               5                   10                  15


<210>    110
<211>    15
<212>    PRT
<213>    Artificial Sequence

<220>
<223>    LMP2a epitope


<400>    110
Ala Val Trp Leu Thr Val Met Thr Asn Thr Leu Leu Ser Ala Trp
1               5                   10                  15


<210>    111
<211>    15
<212>    PRT
<213>    Artificial Sequence

<220>
<223>    LMP2a epitope


<400>    111
Thr Val Met Thr Asn Thr Leu Leu Ser Ala Trp Ile Leu Thr Ala
1               5                   10                  15


<210>    112
<211>    15
<212>    PRT
<213>    Artificial Sequence

<220>
<223>    LMP2a epitope

<400>    112
Asn Thr Leu Leu Ser Ala Trp Ile Leu Thr Ala Gly Phe Leu Ile
1               5                   10                  15

<210>    113
<211>    15
<212>    PRT
<213>    Artificial Sequence

<220>
<223>    LMP2a epitope

<400>    113
Ser Ala Trp Ile Leu Thr Ala Gly Phe Leu Ile Phe Leu Ile Gly
1               5                   10                  15

<210>    114
<211>    15
<212>    PRT
<213>    Artificial Sequence

<220>
<223>    LMP2a epitope

<400>    114
Leu Thr Ala Gly Phe Leu Ile Phe Leu Ile Gly Phe Ala Leu Phe
1               5                   10                  15

<210>    115
<211>    15
<212>    PRT
<213>    Artificial Sequence

<220>
<223>    LMP2a epitope

<400>    115
Phe Leu Ile Phe Leu Ile Gly Phe Ala Leu Phe Gly Val Ile Arg
1               5                   10                  15

<210>    116
<211>    15
<212>    PRT
<213>    Artificial Sequence

<220>
<223>    LMP2a epitope

<400>    116
Leu Ile Gly Phe Ala Leu Phe Gly Val Ile Arg Cys Cys Arg Tyr
1               5                   10                  15

<210>    117

<211>     15
<212>     PRT
<213>     Artificial Sequence

<220>
<223>     LMP2a epitope


<400>     117
Ala Leu Phe Gly Val Ile Arg Cys Cys Arg Tyr Cys Cys Tyr Tyr
1               5                   10                  15


<210>     118
<211>     15
<212>     PRT
<213>     Artificial Sequence

<220>
<223>     LMP2a epitope


<400>     118
Val Ile Arg Cys Cys Arg Tyr Cys Cys Tyr Tyr Cys Leu Thr Leu
1               5                   10                  15


<210>     119
<211>     15
<212>     PRT
<213>     Artificial Sequence

<220>
<223>     LMP2a epitope


<400>     119
Cys Arg Tyr Cys Cys Tyr Tyr Cys Leu Thr Leu Glu Ser Glu Glu
1               5                   10                  15


<210>     120
<211>     15
<212>     PRT
<213>     Artificial Sequence

<220>
<223>     LMP2a epitope


<400>     120
Cys Tyr Tyr Cys Leu Thr Leu Glu Ser Glu Glu Arg Pro Pro Thr
1               5                   10                  15


<210>     121
<211>     15
<212>     PRT
<213>     Artificial Sequence

<220>
<223>     LMP2a epitope

<400>    121
Leu Thr Leu Glu Ser Glu Glu Arg Pro Pro Thr Pro Tyr Arg Asn
1               5                   10                  15

<210>    122
<211>    15
<212>    PRT
<213>    Artificial Sequence

<220>
<223>    LMP2a epitope

<400>    122
Leu Glu Ser Glu Glu Arg Pro Pro Thr Pro Tyr Arg Asn Thr Val
1               5                   10                  15

<210>    123
<211>    15
<212>    PRT
<213>    Artificial Sequence

<220>
<223>    EBNA-1 epitope

<400>    123
Gly Gly Ala Gly Ala Gly Gly Gly Gly Arg Gly Arg Gly Gly Ser
1               5                   10                  15

<210>    124
<211>    15
<212>    PRT
<213>    Artificial Sequence

<220>
<223>    EBNA-1 epitope

<400>    124
Ala Gly Gly Gly Gly Arg Gly Arg Gly Gly Ser Gly Gly Arg Gly
1               5                   10                  15

<210>    125
<211>    15
<212>    PRT
<213>    Artificial Sequence

<220>
<223>    EBNA-1 epitope

<400>    125
Gly Arg Gly Arg Gly Gly Ser Gly Gly Arg Gly Arg Gly Gly Ser
1               5                   10                  15

<210>    126

<211>    15
<212>    PRT
<213>    Artificial Sequence

<220>
<223>    EBNA-1 epitope


<400>    126
Gly Gly Ser Gly Gly Arg Gly Arg Gly Gly Ser Gly Gly Arg Gly
1               5                   10                  15


<210>    127
<211>    15
<212>    PRT
<213>    Artificial Sequence

<220>
<223>    EBNA-1 epitope


<400>    127
Gly Arg Gly Arg Gly Gly Ser Gly Gly Arg Gly Arg Gly Gly Ser
1               5                   10                  15


<210>    128
<211>    15
<212>    PRT
<213>    Artificial Sequence

<220>
<223>    EBNA-1 epitope


<400>    128
Gly Gly Ser Gly Gly Arg Gly Arg Gly Gly Ser Gly Gly Arg Arg
1               5                   10                  15


<210>    129
<211>    15
<212>    PRT
<213>    Artificial Sequence

<220>
<223>    EBNA-1 epitope


<400>    129
Gly Arg Gly Arg Gly Gly Ser Gly Gly Arg Arg Gly Arg Gly Arg
1               5                   10                  15


<210>    130
<211>    15
<212>    PRT
<213>    Artificial Sequence

<220>
<223>    EBNA-1 epitope

<400>    130
Gly Gly Ser Gly Gly Arg Arg Gly Arg Gly Arg Glu Arg Ala Arg
1               5                   10                  15

<210>    131
<211>    15
<212>    PRT
<213>    Artificial Sequence

<220>
<223>    EBNA-1 epitope

<400>    131
Gly Arg Arg Gly Arg Gly Arg Glu Arg Ala Arg Gly Gly Ser Arg
1               5                   10                  15

<210>    132
<211>    15
<212>    PRT
<213>    Artificial Sequence

<220>
<223>    EBNA-1 epitope

<400>    132
Arg Gly Arg Glu Arg Ala Arg Gly Gly Ser Arg Glu Arg Ala Arg
1               5                   10                  15

<210>    133
<211>    15
<212>    PRT
<213>    Artificial Sequence

<220>
<223>    EBNA-1 epitope

<400>    133
Arg Ala Arg Gly Gly Ser Arg Glu Arg Ala Arg Gly Arg Gly Arg
1               5                   10                  15

<210>    134
<211>    15
<212>    PRT
<213>    Artificial Sequence

<220>
<223>    EBNA-1 epitope

<400>    134
Gly Ser Arg Glu Arg Ala Arg Gly Arg Gly Arg Gly Arg Gly Glu
1               5                   10                  15

<210>    135

<211>    15
<212>    PRT
<213>    Artificial Sequence

<220>
<223>    EBNA-1 epitope


<400>    135
Arg Ala Arg Gly Arg Gly Arg Gly Arg Gly Glu Lys Arg Pro Arg
1               5                   10                  15


<210>    136
<211>    15
<212>    PRT
<213>    Artificial Sequence

<220>
<223>    EBNA-1 epitope


<400>    136
Arg Gly Arg Gly Arg Gly Glu Lys Arg Pro Arg Ser Pro Ser Ser
1               5                   10                  15


<210>    137
<211>    15
<212>    PRT
<213>    Artificial Sequence

<220>
<223>    EBNA-1 epitope


<400>    137
Arg Gly Glu Lys Arg Pro Arg Ser Pro Ser Ser Gln Ser Ser Ser
1               5                   10                  15


<210>    138
<211>    15
<212>    PRT
<213>    Artificial Sequence

<220>
<223>    EBNA-1 epitope


<400>    138
Arg Pro Arg Ser Pro Ser Ser Gln Ser Ser Ser Ser Gly Ser Pro
1               5                   10                  15


<210>    139
<211>    15
<212>    PRT
<213>    Artificial Sequence

<220>
<223>    EBNA-1 epitope

<400>    139
Pro Ser Ser Gln Ser Ser Ser Ser Gly Ser Pro Pro Arg Arg Pro
1               5                   10                  15

<210>    140
<211>    15
<212>    PRT
<213>    Artificial Sequence

<220>
<223>    EBNA-1 epitope

<400>    140
Ser Ser Ser Ser Gly Ser Pro Pro Arg Arg Pro Pro Pro Gly Arg
1               5                   10                  15

<210>    141
<211>    15
<212>    PRT
<213>    Artificial Sequence

<220>
<223>    EBNA-1 epitope

<400>    141
Gly Ser Pro Pro Arg Arg Pro Pro Pro Gly Arg Arg Pro Phe Phe
1               5                   10                  15

<210>    142
<211>    15
<212>    PRT
<213>    Artificial Sequence

<220>
<223>    EBNA-1 epitope

<400>    142
Arg Arg Pro Pro Pro Gly Arg Arg Pro Phe Phe His Pro Val Gly
1               5                   10                  15

<210>    143
<211>    15
<212>    PRT
<213>    Artificial Sequence

<220>
<223>    EBNA-1 epitope

<400>    143
Pro Gly Arg Arg Pro Phe Phe His Pro Val Gly Asp Ala Asp Tyr
1               5                   10                  15

<210>    144

<211>     15
<212>     PRT
<213>     Artificial Sequence

<220>
<223>     EBNA-1 epitope


<400>     144
Pro Phe Phe His Pro Val Gly Asp Ala Asp Tyr Phe Glu Tyr Leu
1               5                   10                  15


<210>     145
<211>     15
<212>     PRT
<213>     Artificial Sequence

<220>
<223>     EBNA-1 epitope


<400>     145
Pro Val Gly Asp Ala Asp Tyr Phe Glu Tyr Leu Gln Glu Gly Gly
1               5                   10                  15


<210>     146
<211>     15
<212>     PRT
<213>     Artificial Sequence

<220>
<223>     EBNA-1 epitope


<400>     146
Ala Asp Tyr Phe Glu Tyr Leu Gln Glu Gly Gly Pro Asp Gly Glu
1               5                   10                  15


<210>     147
<211>     15
<212>     PRT
<213>     Artificial Sequence

<220>
<223>     EBNA-1 epitope


<400>     147
Glu Tyr Leu Gln Glu Gly Gly Pro Asp Gly Glu Pro Asp Val Pro
1               5                   10                  15


<210>     148
<211>     15
<212>     PRT
<213>     Artificial Sequence

<220>
<223>     EBNA-1 epitope

<400>    148
Glu Gly Gly Pro Asp Gly Glu Pro Asp Val Pro Pro Gly Ala Ile
1               5                   10                  15


<210>    149
<211>    15
<212>    PRT
<213>    Artificial Sequence

<220>
<223>    EBNA-1 epitope


<400>    149
Asp Gly Glu Pro Asp Val Pro Pro Gly Ala Ile Glu Gln Gly Pro
1               5                   10                  15


<210>    150
<211>    15
<212>    PRT
<213>    Artificial Sequence

<220>
<223>    EBNA-1 epitope


<400>    150
Asp Val Pro Pro Gly Ala Ile Glu Gln Gly Pro Thr Asp Asp Pro
1               5                   10                  15


<210>    151
<211>    15
<212>    PRT
<213>    Artificial Sequence

<220>
<223>    EBNA-1 epitope


<400>    151
Gly Ala Ile Glu Gln Gly Pro Thr Asp Asp Pro Gly Glu Gly Pro
1               5                   10                  15


<210>    152
<211>    15
<212>    PRT
<213>    Artificial Sequence

<220>
<223>    EBNA-1 epitope


<400>    152
Gln Gly Pro Thr Asp Asp Pro Gly Glu Gly Pro Ser Thr Gly Pro
1               5                   10                  15


<210>    153

<211>    15
<212>    PRT
<213>    Artificial Sequence

<220>
<223>    EBNA-1 epitope


<400>    153
Asp Asp Pro Gly Glu Gly Pro Ser Thr Gly Pro Arg Gly Gln Gly
1               5                   10                  15


<210>    154
<211>    15
<212>    PRT
<213>    Artificial Sequence

<220>
<223>    EBNA-1 epitope


<400>    154
Glu Gly Pro Ser Thr Gly Pro Arg Gly Gln Gly Asp Gly Gly Arg
1               5                   10                  15


<210>    155
<211>    15
<212>    PRT
<213>    Artificial Sequence

<220>
<223>    EBNA-1 epitope


<400>    155
Thr Gly Pro Arg Gly Gln Gly Asp Gly Gly Arg Arg Lys Lys Gly
1               5                   10                  15


<210>    156
<211>    15
<212>    PRT
<213>    Artificial Sequence

<220>
<223>    EBNA-1 epitope


<400>    156
Gly Gln Gly Asp Gly Gly Arg Arg Lys Lys Gly Gly Trp Phe Gly
1               5                   10                  15


<210>    157
<211>    15
<212>    PRT
<213>    Artificial Sequence

<220>
<223>    EBNA-1 epitope

<400>    157
Gly Gly Arg Arg Lys Lys Gly Gly Trp Phe Gly Lys His Arg Gly
1               5                   10                  15

<210>    158
<211>    15
<212>    PRT
<213>    Artificial Sequence

<220>
<223>    EBNA-1 epitope

<400>    158
Lys Lys Gly Gly Trp Phe Gly Lys His Arg Gly Gln Gly Gly Ser
1               5                   10                  15

<210>    159
<211>    15
<212>    PRT
<213>    Artificial Sequence

<220>
<223>    EBNA-1 epitope

<400>    159
Trp Phe Gly Lys His Arg Gly Gln Gly Gly Ser Asn Pro Lys Phe
1               5                   10                  15

<210>    160
<211>    15
<212>    PRT
<213>    Artificial Sequence

<220>
<223>    EBNA-1 epitope

<400>    160
His Arg Gly Gln Gly Gly Ser Asn Pro Lys Phe Glu Asn Ile Ala
1               5                   10                  15

<210>    161
<211>    15
<212>    PRT
<213>    Artificial Sequence

<220>
<223>    EBNA-1 epitope

<400>    161
Gly Gly Ser Asn Pro Lys Phe Glu Asn Ile Ala Glu Gly Leu Arg
1               5                   10                  15

<210>    162

<211>    15
<212>    PRT
<213>    Artificial Sequence

<220>
<223>    EBNA-1 epitope


<400>    162
Pro Lys Phe Glu Asn Ile Ala Glu Gly Leu Arg Val Leu Leu Ala
1               5                   10                  15


<210>    163
<211>    15
<212>    PRT
<213>    Artificial Sequence

<220>
<223>    EBNA-1 epitope


<400>    163
Asn Ile Ala Glu Gly Leu Arg Val Leu Leu Ala Arg Ser His Val
1               5                   10                  15


<210>    164
<211>    15
<212>    PRT
<213>    Artificial Sequence

<220>
<223>    EBNA-1 epitope


<400>    164
Gly Leu Arg Val Leu Leu Ala Arg Ser His Val Glu Arg Thr Thr
1               5                   10                  15


<210>    165
<211>    15
<212>    PRT
<213>    Artificial Sequence

<220>
<223>    EBNA-1 epitope


<400>    165
Leu Leu Ala Arg Ser His Val Glu Arg Thr Thr Glu Glu Gly Asn
1               5                   10                  15


<210>    166
<211>    15
<212>    PRT
<213>    Artificial Sequence

<220>
<223>    EBNA-1 epitope

<400>    166
Ser His Val Glu Arg Thr Thr Glu Glu Gly Asn Trp Val Ala Gly
1               5                   10                  15

<210>    167
<211>    15
<212>    PRT
<213>    Artificial Sequence

<220>
<223>    EBNA-1 epitope

<400>    167
Arg Thr Thr Glu Glu Gly Asn Trp Val Ala Gly Val Phe Val Tyr
1               5                   10                  15

<210>    168
<211>    15
<212>    PRT
<213>    Artificial Sequence

<220>
<223>    EBNA-1 epitope

<400>    168
Glu Gly Asn Trp Val Ala Gly Val Phe Val Tyr Gly Gly Ser Lys
1               5                   10                  15

<210>    169
<211>    15
<212>    PRT
<213>    Artificial Sequence

<220>
<223>    EBNA-1 epitope

<400>    169
Val Ala Gly Val Phe Val Tyr Gly Gly Ser Lys Thr Ser Leu Tyr
1               5                   10                  15

<210>    170
<211>    15
<212>    PRT
<213>    Artificial Sequence

<220>
<223>    EBNA-1 epitope

<400>    170
Phe Val Tyr Gly Gly Ser Lys Thr Ser Leu Tyr Asn Leu Arg Arg
1               5                   10                  15

<210>    171

<211>    15
<212>    PRT
<213>    Artificial Sequence

<220>
<223>    EBNA-1 epitope


<400>    171
Gly Ser Lys Thr Ser Leu Tyr Asn Leu Arg Arg Gly Ile Ala Leu
1               5                   10                  15


<210>    172
<211>    15
<212>    PRT
<213>    Artificial Sequence

<220>
<223>    EBNA-1 epitope


<400>    172
Ser Leu Tyr Asn Leu Arg Arg Gly Ile Ala Leu Ala Val Pro Gln
1               5                   10                  15


<210>    173
<211>    15
<212>    PRT
<213>    Artificial Sequence

<220>
<223>    EBNA-1 epitope


<400>    173
Leu Arg Arg Gly Ile Ala Leu Ala Val Pro Gln Cys Arg Ile Thr
1               5                   10                  15


<210>    174
<211>    15
<212>    PRT
<213>    Artificial Sequence

<220>
<223>    EBNA-1 epitope


<400>    174
Ile Ala Leu Ala Val Pro Gln Cys Arg Ile Thr Pro Leu Ser Arg
1               5                   10                  15


<210>    175
<211>    15
<212>    PRT
<213>    Artificial Sequence

<220>
<223>    EBNA-1 epitope

<400>    175
Val Pro Gln Cys Arg Ile Thr Pro Leu Ser Arg Leu Pro Phe Gly
1               5                   10                  15

<210>    176
<211>    15
<212>    PRT
<213>    Artificial Sequence

<220>
<223>    EBNA-1 epitope

<400>    176
Arg Ile Thr Pro Leu Ser Arg Leu Pro Phe Gly Met Ala Pro Gly
1               5                   10                  15

<210>    177
<211>    15
<212>    PRT
<213>    Artificial Sequence

<220>
<223>    EBNA-1 epitope

<400>    177
Leu Ser Arg Leu Pro Phe Gly Met Ala Pro Gly Pro Gly Pro Gln
1               5                   10                  15

<210>    178
<211>    15
<212>    PRT
<213>    Artificial Sequence

<220>
<223>    EBNA-1 epitope

<400>    178
Pro Phe Gly Met Ala Pro Gly Pro Gly Pro Gln Pro Gly Pro Leu
1               5                   10                  15

<210>    179
<211>    15
<212>    PRT
<213>    Artificial Sequence

<220>
<223>    EBNA-1 epitope

<400>    179
Ala Pro Gly Pro Gly Pro Gln Pro Gly Pro Leu Arg Glu Ser Ile
1               5                   10                  15

<210>    180

<211>    15
<212>    PRT
<213>    Artificial Sequence

<220>
<223>    EBNA-1 epitope


<400>    180
Gly Pro Gln Pro Gly Pro Leu Arg Glu Ser Ile Val Cys Tyr Phe
1               5                   10                  15


<210>    181
<211>    15
<212>    PRT
<213>    Artificial Sequence

<220>
<223>    EBNA-1 epitope


<400>    181
Gly Pro Leu Arg Glu Ser Ile Val Cys Tyr Phe Met Val Phe Leu
1               5                   10                  15


<210>    182
<211>    15
<212>    PRT
<213>    Artificial Sequence

<220>
<223>    EBNA-1 epitope


<400>    182
Glu Ser Ile Val Cys Tyr Phe Met Val Phe Leu Gln Thr His Ile
1               5                   10                  15


<210>    183
<211>    15
<212>    PRT
<213>    Artificial Sequence

<220>
<223>    EBNA-1 epitope


<400>    183
Cys Tyr Phe Met Val Phe Leu Gln Thr His Ile Phe Ala Glu Val
1               5                   10                  15


<210>    184
<211>    15
<212>    PRT
<213>    Artificial Sequence

<220>
<223>    EBNA-1 epitope

<400>    184
Val Phe Leu Gln Thr His Ile Phe Ala Glu Val Leu Lys Asp Ala
1               5                   10                  15

<210>    185
<211>    15
<212>    PRT
<213>    Artificial Sequence

<220>
<223>    EBNA-1 epitope

<400>    185
Thr His Ile Phe Ala Glu Val Leu Lys Asp Ala Ile Lys Asp Leu
1               5                   10                  15

<210>    186
<211>    15
<212>    PRT
<213>    Artificial Sequence

<220>
<223>    EBNA-1 epitope

<400>    186
Ala Glu Val Leu Lys Asp Ala Ile Lys Asp Leu Val Met Thr Lys
1               5                   10                  15

<210>    187
<211>    15
<212>    PRT
<213>    Artificial Sequence

<220>
<223>    EBNA-1 epitope

<400>    187
Lys Asp Ala Ile Lys Asp Leu Val Met Thr Lys Pro Ala Pro Thr
1               5                   10                  15

<210>    188
<211>    15
<212>    PRT
<213>    Artificial Sequence

<220>
<223>    EBNA-1 epitope

<400>    188
Lys Asp Leu Val Met Thr Lys Pro Ala Pro Thr Cys Asn Ile Lys
1               5                   10                  15

<210>    189

<211>    15
<212>    PRT
<213>    Artificial Sequence

<220>
<223>    EBNA-1 epitope


<400>    189
Met Thr Lys Pro Ala Pro Thr Cys Asn Ile Lys Val Thr Val Cys
1               5                   10                  15


<210>    190
<211>    15
<212>    PRT
<213>    Artificial Sequence

<220>
<223>    EBNA-1 epitope


<400>    190
Ala Pro Thr Cys Asn Ile Lys Val Thr Val Cys Ser Phe Asp Asp
1               5                   10                  15


<210>    191
<211>    15
<212>    PRT
<213>    Artificial Sequence

<220>
<223>    EBNA-1 epitope


<400>    191
Asn Ile Lys Val Thr Val Cys Ser Phe Asp Asp Gly Val Asp Leu
1               5                   10                  15


<210>    192
<211>    15
<212>    PRT
<213>    Artificial Sequence

<220>
<223>    EBNA-1 epitope


<400>    192
Thr Val Cys Ser Phe Asp Asp Gly Val Asp Leu Pro Pro Trp Phe
1               5                   10                  15


<210>    193
<211>    15
<212>    PRT
<213>    Artificial Sequence

<220>
<223>    EBNA-1 epitope

<400>    193
Phe Asp Asp Gly Val Asp Leu Pro Pro Trp Phe Pro Pro Met Val
1               5                   10                  15

<210>    194
<211>    15
<212>    PRT
<213>    Artificial Sequence

<220>
<223>    EBNA-1 epitope

<400>    194
Val Asp Leu Pro Pro Trp Phe Pro Pro Met Val Glu Gly Ala Ala
1               5                   10                  15

<210>    195
<211>    15
<212>    PRT
<213>    Artificial Sequence

<220>
<223>    EBNA-1 epitope

<400>    195
Pro Trp Phe Pro Pro Met Val Glu Gly Ala Ala Ala Glu Gly Asp
1               5                   10                  15

<210>    196
<211>    15
<212>    PRT
<213>    Artificial Sequence

<220>
<223>    EBNA-1 epitope

<400>    196
Pro Met Val Glu Gly Ala Ala Ala Glu Gly Asp Asp Gly Asp Asp
1               5                   10                  15

<210>    197
<211>    15
<212>    PRT
<213>    Artificial Sequence

<220>
<223>    EBNA-1 epitope

<400>    197
Gly Ala Ala Ala Glu Gly Asp Asp Gly Asp Asp Gly Asp Glu Gly
1               5                   10                  15

<210>    198

<211>    15
<212>    PRT
<213>    Artificial Sequence

<220>
<223>    EBNA-1 epitope


<400>    198
Glu Gly Asp Asp Gly Asp Asp Gly Asp Glu Gly Gly Asp Gly Asp
1               5                   10                  15


<210>    199
<211>    15
<212>    PRT
<213>    Artificial Sequence

<220>
<223>    EBNA-1 epitope


<400>    199
Gly Asp Asp Gly Asp Glu Gly Gly Asp Gly Asp Glu Gly Glu Glu
1               5                   10                  15


<210>    200
<211>    15
<212>    PRT
<213>    Artificial Sequence

<220>
<223>    EBNA-1 epitope


<400>    200
Gly Asp Glu Gly Gly Asp Gly Asp Glu Gly Glu Glu Gly Gln Glu
1               5                   10                  15